Europäisches Patentamt

European Patent Office

Office européen des brevets

(11) Publication number: **0 136 855**
**B1**

(12) EUROPEAN PATENT SPECIFICATION

(45) Date of publication of patent specification: **15.11.89**

(21) Application number: **84306208.4**

(22) Date of filing: **10.09.84**

(51) Int. Cl.⁴: **A 61 B 17/39**

(54) Electrosurgical generator.

(30) Priority: **13.09.83 US 531621**
**13.09.83 US 531758**

(43) Date of publication of application:
**10.04.85 Bulletin 85/15**

(45) Publication of the grant of the patent:
**15.11.89 Bulletin 89/46**

(84) Designated Contracting States:
**AT BE CH DE FR GB IT LI LU NL SE**

(56) References cited:
**EP-A-0 126 814**
**DE-A-2 803 017**
**DE-A-3 120 102**
**FR-A-2 474 307**
**US-A-1 995 526**

(73) Proprietor: **VALLEYLAB, INC.**
**5920 Longbow Drive**
**Boulder Colorado 80301 (US)**

(72) Inventor: **Harris, Frank Werner**
**640 Yale Road**
**Boulder Colorado (US)**
Inventor: **Hulett III, Frederic Marshall**
**2605 South Endora**
**Denver Colorado (US)**

(74) Representative: **Boydell, John Christopher et al**
**Stevens, Hewlett & Perkins 5 Quality Court**
**Chancery Lane**
**London, WC2A 1HZ (GB)**

Courier Press, Leamington Spa, England.

**Description**

This invention relates to electrosurgical generators and in particular to such generators including means for controlling the output power level thereof. The invention further relates to the use of such generators with bipolar forceps or handpieces.

Bipolar electrosurgery refers to the use of a handpiece having two small electrodes to apply electrosurgical current instead of a single small active and a large return electrode as is used in monopolar electrosurgery.

The advantages of bipolar electrosurgery over monopolar are:—

(1) A lower power level is used which translates directly to less tissue destruction.

(2) The only tissue destroyed is that located between the bipolar electrodes so that there is virtually no danger of alternate site burns.

(3) The applied voltage can be much lower. This prevents tissue charring and scarring due to sparks at the electrodes.

Bipolar electrosurgery is used extensively in surgical procedures on the eye and brain where the delicate tissue can be easily damaged by excessive heat or sparking.

In an effort to improve the effects of bipolar electrosurgery, studies have been conducted on the variation of tissue impedance during desiccation. The results showed two phases of desiccation. The first phase begins as soon as electrosurgical power is applied. The tissue temperature rises, cell walls are ruptured and the impedance of the tissue shows a decrease. The temperature continues to rise and the water is driven off as steam. As tissue dries out, the resistance rises. The output voltage goes up somewhat as the resistance rises and at this point sparking, excessive heating of the forceps, or sticking of forceps to tissue may occur.

According to a first aspect of the invention there is provided an electrosurgical generator comprising:—

a source of electrosurgical RF energy;

means for connecting said source to a patient;

sensor means connected to said RF source for sensing the patient impedance; and

control means connected to said RF source for controlling the output power from said RF source in accordance with said patient impedance, said control means being operable to decrease the output power from said RF source with increasing patient impedance, characterized in that the rate of RF output power decrease is substantially greater than that which would result if the output voltage from the RF source were maintained constant over the range of increasing patient impedance.

Thus it will be seen that the power diminishes towards the end of the desiccation stage.

Preferably the arrangement is such that the power decreases as the inverse of the square of the patient's tissue impedance.

According to a second aspect of the invention there is provided an electrosurgical generator comprising:—

an adjustable source of electrosurgical RF energy;

means for connecting said source to a load including a patient; and

control means for adjusting the power delivered to said load from said source, said control means including means for selecting a plurality of modes of operation of said generator in accordance with said load impedance, said generator being characterised in that said control means includes:—

means for selecting first and second modes of operation of said RF source when said load impedance exceeds a predetermined value;

means for maintaining the voltage across said load substantially constant for all values of said load impedance in excess of said predetermined value in response to said first mode being selected; and

means for reducing the RF output power delivered to said load in accordance with the square of the load impedance for all values of load impedance in excess of said predetermined value in response to said second mode being selected.

Preferably the arrangement is such that, for impedance above a predetermined value, one of two different modes of operation may be selected. Preferably one of the selected modes provides a constant voltage characteristic, and the other the characteristic described above where the power is decreased in accordance with the square of the load impedance.

In a preferred embodiment, the generator is operable to compute a required power for different load impedance ranges, and includes circuitry for conforming the actual output power to the computed required power.

U.S. patents 3946487; 3980085; 4126137; 4188927; 4474179, and 4092986 disclose means for reducing the output current in accordance with increasing load impedance. In particular, these patents teach the use of constant voltage outputs whereby the current is decreased with increasing load impedance. However, there is no disclosure in these patents that output power be inversely varied in accordance with the square of the load impedance. In general, there is no disclosure that output power be decreased at a rate which is substantially greater than that which results on the constant voltage outputs of these patents.

In order that the invention may be better understood an embodiment thereof will now be described by way of example only and with reference to the accompanying drawings in which:—

2

Figure 1 is a block diagram of an embodiment of an electrosurgical generator in accordance with the invention;

Figure 2 is a graph illustrating different modes of operation of the generator of the present invention;

Figure 3 is a flow chart of the program executed by and stored in the microprocessor controller of Figure 1; and

Figures 4A and 4B are a schematic diagram of the switching power supply, the generator and the voltage and current sensing circuitry of Figure 1.

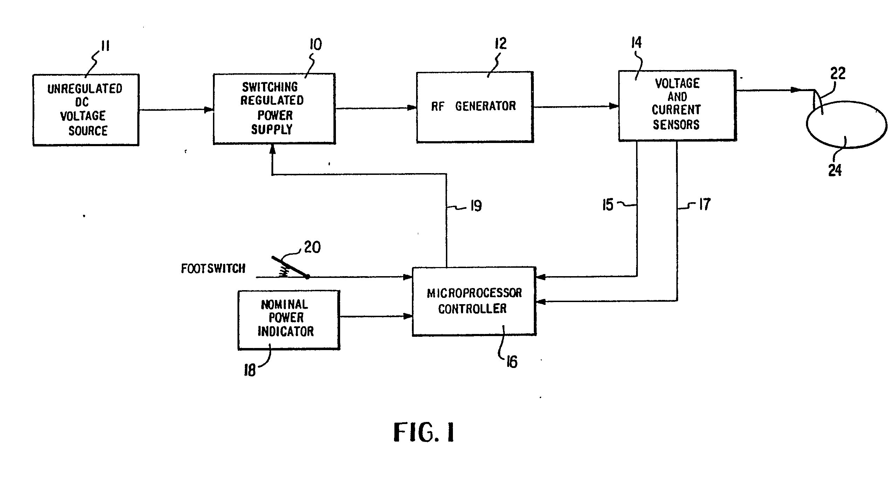

Referring to Figure 1, an illustrative electrosurgical generator in accordance with the invention includes a switching regulated power supply 10, an unregulated DC voltage source 11, an RF generator 12, voltage and current sensors 14, a microprocessor controller 16, a nominal power indicator 18, and a foot-switch 20. Electrosurgical current is applied from the generator to forceps 22, which are diagramatically shown in contact with a patient 24.

The power delivered to the load is a function of the voltage from DC supply 10 and the load impedance. As will be described in more detail hereinafter, sensors 14 develop sensing signals proportional to the load voltage and current which are respectively applied over lines 15 and 17 to controller 16. The controller digitizes the sensing signals and computes the load impedance and the actual power being delivered to the load.

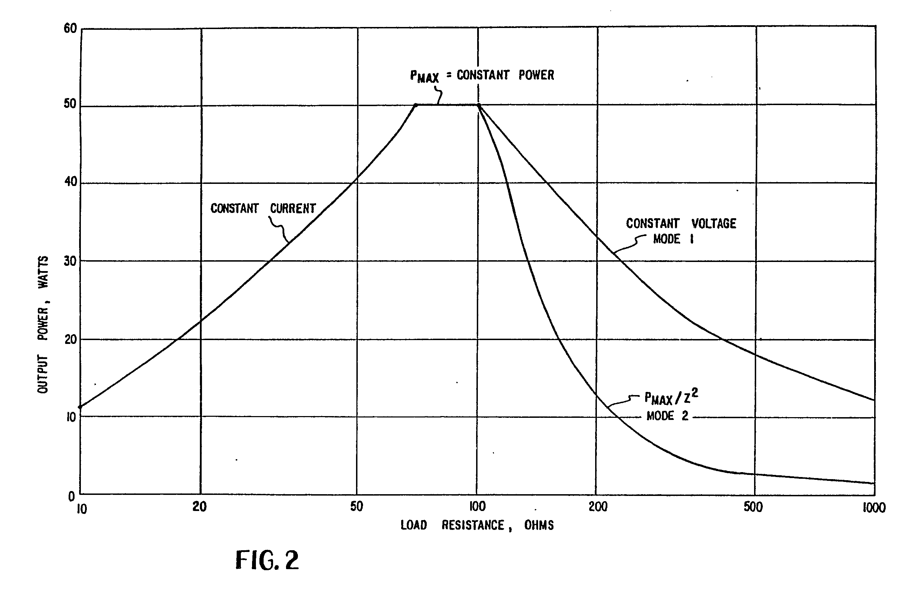

A required load power is also calculated which is a function of the calculated load impedance and the nominal power selected by the operator at 18. A graph of the required load power for a nominal power of 50 watts is illustrated in Figure 2.

At low load impedances less than typically 70 ohms, the computed required power is reduced to prevent damage or overheating of the RF output. In particular, the control voltage applied over line 19 to supply 10 controls the output power in such a manner as to maintain the load current constant over this low value impedance range. Over a mid range extending from about 70 to 100 ohms, the computed required power is held constant at the selected nominal power.

At impedances above 100 ohms, one of two modes may be chosen by the operator. As indicated in Figure 2, the computed required power is reduced in the first of these modes with the voltage being held constant, this effect being similar to that of known generators. In the second mode the computed required power is reduced at a rate which is substantially greater than that which occurs when the voltage is held constant. Preferably, the computed required power is reduced, in the second mode, as the square of the load impedance. Thus, for example, the computed required power for a load having impedance of 200 ohms is one-fourth that required for a load impedance of 100 ohms. When the impedance is increased to about 800 ohms, for example, in the second mode of operation, a constant voltage characteristic may be implemented since at this level, the power levels may become impractically small.

After the required power has been calculated for a given nominal power and load impedance range, a comparison is then effectively made between the required power and the actual power to adjust the control voltage applied to line 9 by the correct amount to cause the actual power to match the required power.

The load impedance varies continuously, during electrosurgery due to differing amounts of tissue contact, tissue heating, etc. The microprocessor controller 16 accordingly repeats the measurement, calculation and correction process approximately every 20 milliseconds as long as the foot switch 20 or bipolar handswitch (not shown) is closed.

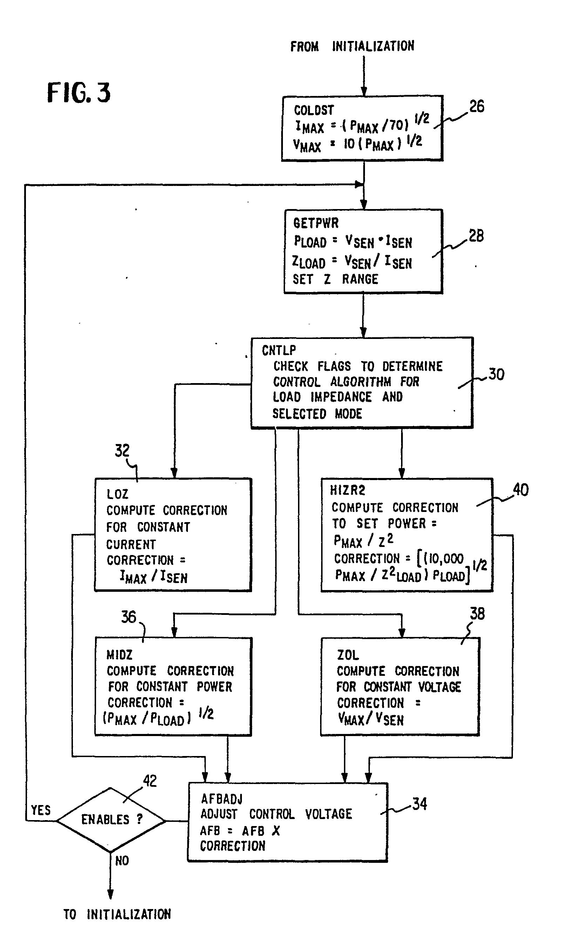

Reference should now be made to Figure 3, which is a generalized flow chart of the program executed by microprocessor controller 16 where the microprocessor may be an INTEL 8039, a member of the 8048 family of single-chip microcomputers and where the program may be stored on an INTEL 2716 programmable memory. After certain initialization routines (not shown), program control pases to routine COLDST 26 (COLD START), which calculates certain parameters and initiates certain functions. First it calculates $I_{MAX}$, which is the current value which should occur at 70 ohms for a given nominal power selected by the operator. For example, if the operator selects a nominal power of 70 watts, the current delivered to a 70 ohm load will be 1 ampere and thus $I_{MAX}$ is 1 ampere in this case. The nominal power range available to the operator typically extends from 0 to 70 watts. The nominal power selected by the operator is termed $P_{MAX}$ and occurs over the mid range impedance extending from 70 to 100 ohms. Since the maximum power occurs over the 70 to 100 ohm range, and since the largest current which will occur in this range occurs at 70 ohms, $I_{MAX}$ is calculated at 70 ohms as described above. In general, $I_{MAX}=(P_{MAX}/70)^{1/2}$ where $P_{MAX}$ equals the nominal power selected by the operator and 70 corresponds to a 70 ohm load.

COLDST also calculates the intial parameter $V_{max}$ in the following manner. Assume the operator selects a nominal power of 25 watts. The voltage occuring across a 100 ohm load for this wattage corresponds to $V_{MAX}$, thus in this case $V_{MAX}$ would be 50 volts. As stated above $P_{MAX}$ occurs over the 70 to 100 ohm range. Further, the maximum voltage will occur with a 100 ohm load. Accordingly, $V_{MAX}$ is calculated in the foregoing manner. In general, $V_{MAX}=(100\ P_{MAX})^{1/2} =10(P_{MAX})^{1/2}$.

After the calculations of $V_{MAX}$ and $I_{MAX}$ COLDST applies a small control voltage AFB over line 19 to supply 10 to turn on RF generator 12. It then waits for about 0.01 second to allow the RF output to become stable before transferring a control to the control routines, GETPWR 28 and CNTLP 30. GETPWR detects the load voltage signal $V_{SEN}$ occuring at line 15 and the load current signal $I_{sen}$ occurring at line 17. The load power $P_{LOAD}$ is calculated as $V_{SEN}I_{SEN}$. Furthermore, the load impedance $Z_{LOAD}$ is calculated as $V_{SEN}/I_{SEN}$. A determination is also made as to which impedance range the load impedance occurs in. Thus, a first impedance range flag (not shown) is set if the load impedance range is less than 70 ohms, a second flag is set if it is between 70 and 100 ohms, and a third flag is set if it is above 100 ohms. It should be understood a substantial amount of tolerance may be employed in the selection of the impedance range may extend from 60 to 116 ohms, for example.

As stated above the operator may select between one of two different modes in the high impedance range. Depending upon the selected mode, a further flag wil be set by COLDST indicating the selected mode. The routine CNTLP inspects the above flags and determines which of the following algoritmms should be executed. These algorithms are LOZ 32, MIDZ 36, ZOL 38, and HIZR2 40 as can be seen in Figure 3. All of these algorithms eventually pass control to a routine AFBADJ 34. Each of these routines will now be individually discussed.

Assuming the load impedance is measured as 40 ohms, CNTLP will transfer control to LOZ 32. A correction factor will be computed which will implement the constant current characteristic described above for the low impedance range. In particular, if $I_{SEN}=1.2$ amps and if $I_{MAX}=1$ amp (for example), then the correction factor is computed as $I_{MAX}/I_{SEN}$. Thus, the calculated factor equals 1.0/1.2. After calculation of the correction factor control is passed to AFBADJ 34 which raises or lowers the control voltage AFB based on the computed correction factor. Thus, if the value of AFB is 10 volts, it will be reduced by the correction factor of 1.0/1.2. In this manner the current is maintained constant at the value of $I_{MAX}$ (1.0 amp) over the low impedance range. Note in this example that $I_{SEN}$ exceeds $I_{MAX}$. Whether it exceeded it or not, the correction factor is calculated as $I_{MAX}/I_{SEN}$ over the entire low impedance range by the LOZ routine.

After the corrected control voltage is applied over line 19 to supply 10, a test is made to determine if the operator is still enabling the generator to apply electrosurgical power to the forceps. This test is conducted at 42. If the generator is so enabled, control is returned to GETPWR. Execution of the routines from GETPWR through AFBADJ requires at most about 20 milliseconds and thus the power delivered to the load is constantly being adjusted depending upon the sensed load conditions. If there are no more enables, the routine may return to an initialization mode where the output is set off and certain self-test routines are executed while waiting for the next enable. This feature is not shown in the flow chart of Figure 3.

As stated above the impedance of the tissue will tend to increase as desiccation proceeds. Hence, assume the next load measure by the GETPWR routine is 80 ohms. the MIDZ flag would be set and the load power calculated. The routine CNTLP would again determine the actuated flags and transfer control to the MIDZ routine 36. There a correction factor is calculated in accordance with the expression $(P_{MAX}/P_{LOAD})^{1/2}$ where $P_{MAX}$ equals the nominal power selected by the operator and $P_{LOAD}=V_{SEN}I_{SEN}$ as calculated in GETPWR. Thus, if $P_{LOAD}$, the actual load power, is 60 watts and $P_{MAX}$ the nominal power is 70 watts, the calculated correction factor is $(7/6)^{1/2}$. Once this correction factor has been determined, control is passed to the AFBADJ routine 34 where the correction factor is multiplied by the previous value of control voltage AFB applied to line 19. The adjustment of the control voltage and the application over line 19 is executed in the same manner as described above for the LOZ routine.

With the passage of further time the impedance continues to increase. Thus, assuming the desiccation mode is still enabled by the operator, control will be returned to GETPWR and CNTLP. Further, assuming the operator has selected mode one for the high range impedances, CNTLP 30 will transfer control to ZOL 38 after having sensed the appropriate flags. If the impedance calculated by GETPWR is now 200 ohms, the power delivered to it will have a constant voltage characteristic as described above with respect to Figure 2. In particular, the constant voltage will be $V_{MAX}$ as calculated by the COLDST routine. In order to implement this constant voltage characteristic ZOL calculates a correction factor determined by the expression $V_{MAX}/V_{SEN}$ where $V_{SEN}$ equals the load voltage sensed by GETPWR. If the sensed voltage is 70 volts while $V_{MAX}$ is 50 volts, the correction factor will be 5/7. AFBADJ utilizes this correction factor in the same manner as discussed above for LOZ and MIDZ to adjust the control voltage applied to line 19.

If the operator has selected mode 2, this will be sensed by the CNTLP together with the fact that the impedance is now in the high impedance range. Assume that the impedance at this time is 200 ohms while $P_{MAX}$ is 50 watts and $P_{LOAD}=60$ watts. The correction factor is computed as $[(10,000\ P_{MAX}/Z^2_{LOAD})/P_{LOAD}]^{1/2}$. Thus in the above instance, it equals $[(10,000)(50)/(40,000)/60]^{1/2}=(5/24)^{1/2}$. Again, after computation of the correction, control is passed to AFBADJ to adjust the line 19 control voltage. Attached hereto as Appendix I is a program listed for the Figure 3 flowchart where the listing also includes various initialization, testing, and error programs.

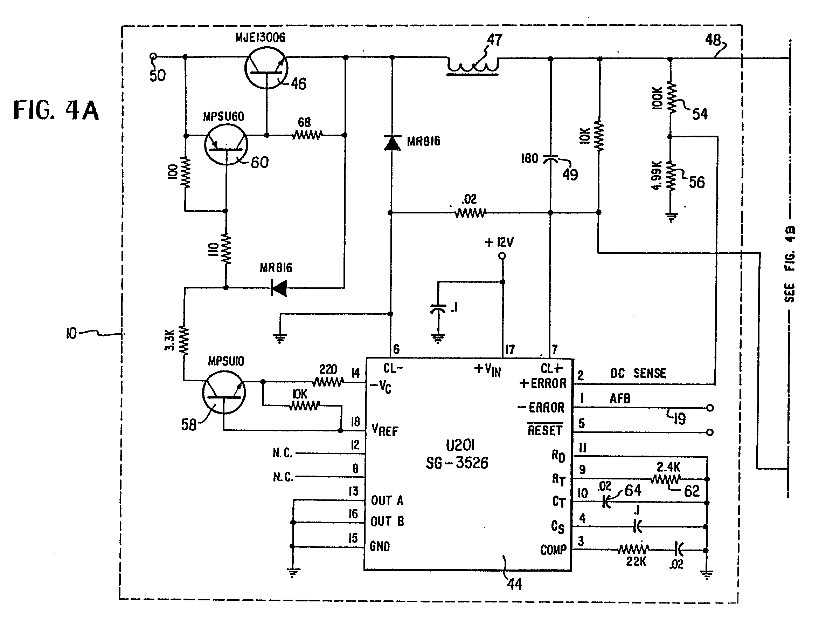

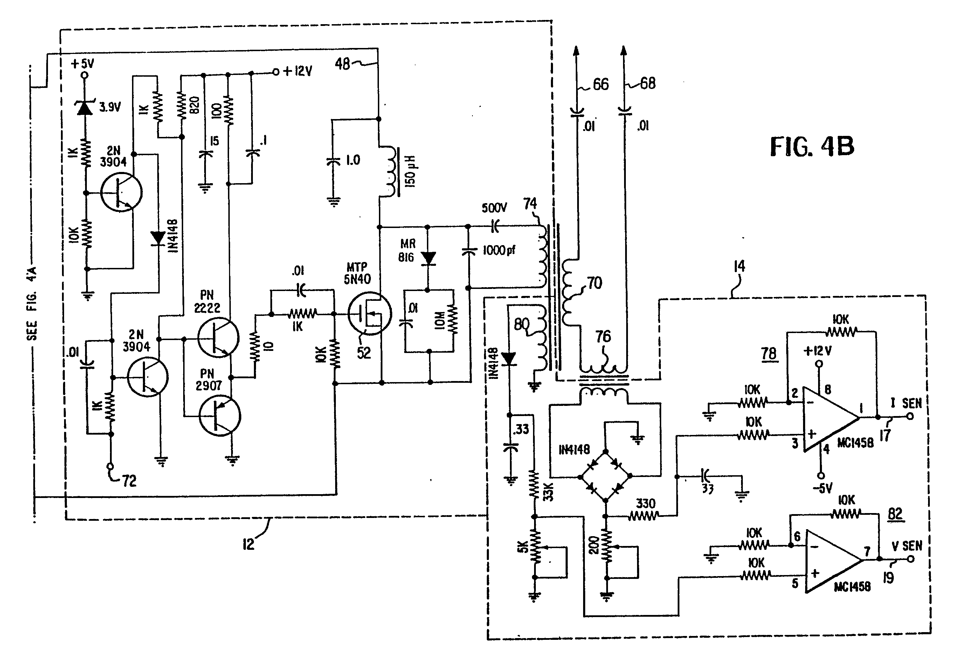

Reference should now be made to Figures 4A and 4B which is a schematic diagram of switching power supply 120, high efficiency RF generator 12, and current sensors 14 of Figure 1. The switching power supply includes a standard switching supply circuit 44 made by Silicon General. The remaining integrated circuits of the invention are also made by Silicon General and other such companies. The switching power supply is of conventional configuration and includes a switching transistor 46 and inductor 47, and an output line 48 which applies regulated high voltage to output transistor 52 of generator 12. The power supply voltage applied to line 48 originates from terminal 50, this voltage typically being about 100 volts. The voltage at terminal 50 is regulated by switching transistor 46, inductor 47 and capacitor 49 where, in particular, the longer transistor 46 is switched off, the less the DC voltage applied to line 48 will be in magnitude. A voltage DC SENSE proportional to the line 48 voltage is applied to terminal 2 of circuit 44 via a voltage divider comprising resistors 54 and 56. This voltage is compared to and follows on line 19.

On-off pulses are applied to switching transistor 46 from circuit 44 via transistors 58 and 60. The repetition rate of these pulses is determined by resistor 62 and capacitor 64 connected to the $R_T$ and $C_T$ terminals of circuit 44. The width of these pulses and thus the on/off time of transistor 46 is a function of the difference between AFB and DC SENSE. Thus, in this manner the power supply voltage follows AFB as the impedance changes during a desiccation procedure.

The output power is delivered to the load via lines 66 and 68 which in turn are connected to output transformer 70. The generator 12 is driven by a 750 KHz signal applied to terminal 72 where it is amplified and the applied to output transistor 52 which in turn drives primary winding 74 of transformer 70.

The load current is sensed at transformer 76 and converted to a voltage representative of the current by the conversion circuitry indicated at 78. This voltage is applied over line 17 and the $I_{SEN}$ signal of the microprocessor 16. Furthermore, a voltage representative of the load voltage is derived from winding 80 of transformer 70. This signal is converted by signal conversion circuitry 82 to develop the load voltage at $V_{SEN}$ on line 19.

The values given for the resistors are in ohms while those for the capacitances are microfarads unless otherwise specified, these values being illustrative of the embodiment of the invention.

It should be noted that overall operation of the present invention is such that a very small heat sink is needed to dissipate heat, thus, a 3—4 watt sink connected to transistor 52 is suitable, such a sink being very small in size. This follows from the fact that the amplifier is essentially matched to a 100 ohm load and only in a limited impedance range around that value is the maximum delivered to the load.

With the load impedance substantially removed from 100 ohms, the power is reduced as discussed above and shown in Figure 2. There is no need to generate substantial wattage at impedances removed from the impedance to which the amplifier is matched. Accordingly, even at impedances removed from 100 ohms only a small amount of power is involved whereby the small heat sink mentioned above is suitable for these smaller powers.

It is to be understood that the above-detailed description of an embodiment of the invention is provided by way of example only. thus, for example, the above principles are also applicable to monopolar as well as bipolar electrosurgery.

APPENDIX I

| LINE | ADDR | B1 B2 B3 B4 | SSE4 BIPOLAR CONTROL:22 NOVEMBER 1982:CHECKSUM C70 | | |
|------|------|-------------|------|------|------|
| 1 | | | TITLE | SSE4 BIPOLAR CONTROL:22 NOVEMBER 1982:CHECKSUM C700 | |
| 2 | | | * | | |
| 3 | | | * | TABLE SETS UP STORAGE LOCATIONS FOR RAM | |
| 4 | | | * | | |
| 6 | | | NLIST | M | |
| 7 | | | ORG | 20H | |
| 8 | 0020 | | ECON | DS | 1 |
| 9 | 0021 | | VSEN | DS | 1 |
| 10 | 0022 | | ISEN | DS | 1 |
| 11 | 0023 | | UCSEN | DS | 1 |
| 12 | 0024 | | Z | DS | 1 | IMPEDANCE OF LOAD.3.125OHMS/COUNT |
| 13 | 0025 | | ZRANGE | DS | 1 |
| 14 | 0026 | | POWER | DS | 1 |
| 15 | 0027 | | PREO | DS | 1 | COMPUTED NECESSARY POWER |
| 16 | 0028 | | AF6 | DS | 1 |
| 17 | 0029 | | CORECI | DS | 1 | CORRECTION FACTOR ON POWER |
| 18 | 002A | | RTECON | DS | 1 |
| 19 | 002B | | IMAX | DS | 1 |
| 20 | 002C | | VMAXR2 | DS | 1 |
| 21 | 002D | | PIMAGE | DS | 1 | PORT 1 IMAGE |
| 22 | 002E | | DWNCNT | DS | 1 | COUNT OF   OF CONSECUTIVE DOWN COUNTS |
| 23 | 002F | | MODES | DS | 1 | KEYING INPUTS G+DSE+DS1F,DS2E |
| 24 | 000B | | DAC | EQU | 05H |
| 25 | 0000 | | CHECK | EQU | R5 |
| 26 | 002B | | VMAXR1 | EQU | 1MAX |
| 27 | 0000 | | COUNT | EQU | R3 |
| 28 | 0008 | | DOG | EQU | 8 |
| 29 | 0001 | | KEY | EQU | 1 |
| 30 | 0002 | | RESET | EQU | 2 |
| 31 | 0004 | | SDN | EQU | 4 |
| 32 | 0010 | | IFLAG | EQU | 16 |
| 33 | 0020 | | VFLAG | EQU | 32 |
| 34 | 0040 | | MAXD | EQU | 64 |
| 35 | 0080 | | ERLITE | EQU | 128 |

```
LINE  ADDR  B1 B2 B3 B4  SSE4 BIPOLAR CONTROL:22 NOVEMBER 1982:CHECKSUM C70


37                      *
38                      *        MACRO DEFINITIONS FOLLOW:
39                      *        RECALL GETS FROM RAM STORAGE AN 8 BIT VALUE
40                      *        AND LOADS IT IN ACCUMULATOR.
41                      *        IF P=0, NOTHING ELSE IS DONE.
42                      *        IF P=1, THEN THE VALUE IS MOVED TO REGISTER RP.
43                      *        FORMAT:
44                      *                 RECALL   ECON,0
45                      *        OR
46                      *                 RECALL   VSEN,1,R4
47                      *
48       RECALL  MACRO  VAL,P,RP
49               MOV    R1, * VAL
50               MOV    A,@R1
51               IF     P
52               MOV    RP,A
53               ENDIF
54               ENDM
```

LINE  ADDR  B1 B2 B3 B4   SSE4 BIPOLAR CONTROL:22 NOVEMBER 1982:CHECKSUM C70

```
                   ********************
                   *
                   *    MACRO STORE
                   *    TAKES A VALUE FROM (1) THE ACCUMULATOR IF R=0
                   *
                   *    OR FROM THE REGISTER RP IF R=1
                   *    AND STORES IT IN LOCATION VAL.
                   *
                   *    FORMAT:
                   *              STORE    ISEN,0
                   *    OR
                   *              STORE POWER,1,K5
                   *
                   *


          STORE MACRO     VAL,R,RP
                MOV       R1,*VAL
                IF        R
                MOV       A,RP
                ENDIF
                MOV       @R1,A
                ENDM
                   *
                   *    MACRO ROUND  ROUNDS UP THE ACCUMULATOR BASED ON MSB OF
                                                                    REGISTER
                   *    FORMAT
                   *              ROUND    RA
                   *
          ROUND MACRO     RX
                XCH       A,RX
                RLC       A         CARRY-ROUNDUP
                CLR       A
                ADDC      A,RX
                ENDM
```

LINE  ADDR  B1 B2 B3 B4  SSE4 BIPOLAR CONTROL:22 NOVEMBER 1982:CHECKSUM C70

```
            *****************************

            *

            *        ADC IS A MACRO WHICH

            *        TAKES CARE OF THE A TO D CONVERSION

            *        AND STORAGE FOR THE ANALOG INPUTS.

            *

            *        FORMAT: ADC        ECON

            *

            ****************************

            ADC    MACRO    WHAT

                   LOCAL    WHERE

                   LOCAL    WHO

                   MOV      R1, WHAT-20H

                   MOV      R0, WHAT

                   MOVX     @R1,A        START CONVERSION

                   MOV      R3,#8

            WHO    DJNZ     R3,WHO       WAIT FOR EDC TO GO DOWN

                   JNI      WHERE

                   JMP      S-2          WAITING FOR INTERRUPT

            WHERE  MOVX     A,@R1

                   MOV      @R0,A        STORE THE RESULT

                   ENDM


            *****************************

            *

            *        WAIT IS A SIMPLE DELAY LOOP

            *        USING REGISTERS 3 AND 4

            *

            *        CALLED BY THE COMMAND

            *

            *        WAIT    N

            *        WHERE N IS ANY INTEGER LESS THAN 256

            *        WILL DELAY 256*2*N MACHINE CYCLES.

            *****************************
```

```
WAIT    MACRO    N
        IF       N
        MOV      R4,#N
        DJNZ     R3,S
        DJNZ     R4,S-Z
        ELSE
        NOP
        NOP
        NOP
        NOP
        NOP
        NOP
        ENDIF
        ENDM
```

LINE  ADDR  B1 B2 B3 B4  SSE4 BIPOLAR CONTROL:22 NOVEMBER 1982:CHECKSUM C70

```
                     *********************************

                     *

                     *

                     *      THIS IS A SIMPLE MINDED ROUTINE

                     *      WHICH RESETS THE WATCHDOG TIMER.

                     *

                     *********************************


       WCHDOG    MACRO

                 ORL      P1,#DOG

                 WAIT     2

                 ANL      P1,#255-DOG    WCHDOG-0

                 WAIT     2

                 ENDM

                     *********************************

                     *

                     *      ROM CHECKSUM ROUTINE

                     *

                     *      THIS ROUTINE SUMS UP ALL POSSIBLE ROM LOCATIONS

                     *      AND IF THE ANSWER IS NOT ZERO, JUMPS OUT TO

                     *      ERROR:  WHICH HANGS UP THE PROCESSOR.

                     *

                     *********************************


       ROMCHK    MACRO    Q,R

                 LOCAL    LOOPA

                 MOV      COUNT,#00

       LOOPA     MOV      A,COUNT

                 MOVP     A,@A

                 ADD      A,CHECK

                 MOV      CHECK,A

                 DJNZ     COUNT,LOOPA

                 IF       Q-1

                 JMP      R       NEXT ROMCH
```

```
                        ELSE
                        RET
                        ENDIF
                        ENDM
        **********************************
        *
        *       SUBTR
        *
        *       SUBTRACTS A REGISTER FROM
        *       THE ACCUMULATOR IN 2S COMPLEMENT
        *       NOTATION
        *       CARRY SET IF ANSWER IS POSITIVE
        *
        **********************************
SUBTR   MACRO   RX
        LOCAL   WHO
        XCH     A,RX
        JNZ     WHO
        INC     A
        INC     RX
WHO     CPL     A
        INC     A           2S COMPLEMENT A
        ADD     A,RX
        ENDM
```

LINE  ADDR  B1 B2 B3 B4  SSE4 BIPOLAR CONTROL:22 NOVEMBER 1982:CHECKSUM C70

```
                         ************************

                         *

                         *     PORT MACRO

                         *     WRITES TO PORT ONE, WITH THE

                         *     VALUE OF KEY, SHUTDOWN OR RESET DESIRED.

                         *

                         *         FORMAT

                         *         PORT      KEY,1

                         *         OR,

                         *         PORT      SDN,0

                         *

                         **************************


         PORT            MACRO     WHAT,N

                         RECALL    PIMAGE,0

                         IF        N

                         ORL       A,#WHAT

                         ELSE

                         ANL       A,#255-WHAT

                         ENDIF

                         OUTL      P1,A

                         STORE PIMAGE,0

                         ENDM

         *ENDMACROS*
```

LINE   ADD   B1 B2 B3 B4   SSE4 BIPOLAR CONTROL:22 NOVEMBER 1982:CHECKSUM C70

```
                      ****************************

                      *

                      *     INITIALIZATION

                      *

                      *     CLEAR THE OUTPUTS AND DAC

                      *     RESET THE WATCHDOG

                      *     RUN ROMCHK AND RAMCHK

                      *     AND LOOK FOR KEYING INPUTS

                      *

                      ****************************

                      *

                      *

                         ORG      00

      0000 75    INIT   ENTO     CLR

      0001 15           DIS      I

      0002 B9 08        MOV      R1,#08H

      0004 27           CLR      A

      0005              STORE    AFB,0

      0008 91           MOVX     @R1,A        CLEAR THE DAC

      0009              WCHDOG

      0019              PORT     KEY,1

      0022              PORT     SDN,0        NO KEY, SHUTDOWN

      0028              PORT     RESET,G      RESETNOT IS ASSERTED HERE
```

LINE ADDR B1 B2 B3 B4    SSE4 BIPOLAR CONTROL:22 NOVEMBER 1982:CHECKSUM C70

```
337                      ••••••••••••••••••••••••••••
338                      •
339                      •    RAMCHK ROUTINE
340                      •
341                      •    THIS ROUTINE WRITES 00,THEN FFH IN R0 TO CHECK IT
342                      •
343                      •    THEN WRITES 00 THEN FFH TO EACH RAM LOCATION IN SUCCESSION.
344                      •    IF A READ OR WRITE IS NOT ACCOMPLISHED,
345                      •    THE ROUTINE JUMPS OUT TO ERROR
346                      •    WHICH SHUTS DOWN ALL PORTS AND HANGS THE PROCESSOR.
347                      •
348                      ••••••••••••••••••••••••••••••••


350  0034  B5 FF    RAMCHK  MOV    R0,#0FFH    ALL IS TO R0
351  0036  F8               MOV    A,R0
352  0037  37              .CPL    A
353  0038  C6 3C            JZ     S+4
354  003A  44 23            JMP    ERROR       ALL IS ERROR
355  003C  A8               MOV    R0,A
356  003D  F8               MOV    A,R0
357  003E  C6 42            JZ     S+4
358  0040  44 23            JMP    ERROR
359  0042  B8 7F            MOV    R0,#07FH    TOP RAM LOCATION
360  0044  37               CPL    A
361  0045  A0       LOOP6   MOV    @R0,A       ALL ONES IN A
362  0046  F0               MOV    A,@R0
363  0047  37               CPL    A           A NOW HAS ALL ZEROS
364  0048  C6 4C            JZ     S+4
365  004A  44 23            JMP    ERROR
366  004C  A0               MOV    @R0,A
367  004D  F0               MOV    A,@R0
368  004E  C6 52            JZ     S+4
369  0050  44 23            JMP    ERROR
370  0052  37               CPL    A           ALL ONES BACK IN A
```

```
371   0053  E8 45        DJNZ    RO,LOOP6    DEC RAM LOC AND CONTINUE
372   0055  23 01        MOV     A,#0001B    SDN,RESETNUT,KEYNOT
373   0057              STORE    PIMAGE,0    RESTORE PIMAGE TO INITIAL CDX
379   005A  39           OUTL    PI,A        AND WRITE TO PORT
```

```
LINE  ADDR  B1 B2 B3 B4  SSE4 BIPOLAR CONTROL:22 NOVEMBER L982:CHECKSUM C70


381                       *
382                       *      THIS IS A SEQUENCE OF ROMCHECK
383                       *      ROUTINES, ONE FOR EACH PAGE OF MEMORY.
384                       *
385                       *      NOW THE CALLUP FOR THE MAIN ROMCHK ROUTINE.
386                       *
387                       *
388   005B  23 00   ROM    MOV     A,#00
389   005D  AD             MOV     CHECK,A
390   005E  04 61          JMP     LOOPB
391   0060  AD             DB      0ADH        THIS IS THE CHECKSUM CORRECTOR
392   0061  14 F0   LOOPB  CALL    ROMST
393   0063  C6 65   ENDINT JZ      COLDST      EVERYTHING OK HERE
394   0065  C4 B2          JMP     KILL6       IF CHECKSUM NOT ZERO
395                       *      NOW DO ALL THE LITTLE ROMCHECKS.
396                       *
397                       *
398                          ORG     0F0H
399   00F0         ROMST   ROMCHK  0,ROM1
411                          ORG     01F4H
412   01F4         ROM1    ROMCHK  0,ROM2
424                          ORG     02F0H
425   02F0         ROM2    ROMCHK  0,ROM3
437                          ORG     03F4H
438   03F4         ROM3    ROMCHK  0,ROM4
450                          ORG     04F0H
451   04F0         ROM4    ROMCHK  0,ROM5
463                          ORG     05F0H
464   05F0         ROM5    ROMCHK  0,ROM6
476                          ORG     06F0H
477   06F0         ROM6    ROMCHK  0,ROM7
489                          ORG     07F0H
490   07F0         ROM7    ROMCHK  1,ROM8      BACK TO MAIN ROUTINE
```

```
LINE  ADDR  B1 B2 B3 B4  SSE4 BIPOLAR CONTROL:22 NOVEMBER 1982:CHECKSUM C70

503                       ****************************
504                       *
505                       *     COLDST ROUTINE
506                       *
507                       *     WHICH BRINGS THE MACHINE UP FROM A COLD START
508                       *     TO AN INITIAL VALUE OF 5 VOLTS
509                       *****************************

511                              ORG    ENDINT+2
512   0065          COLDST       WCHDOG
541   0075  B5                   CLR    F0          RANGE 1 SETUP
542   0076  B8 06                MOV    R0,#DAC     SET DAC VOLTS TO ZERO
543   0078  27                   CLR    A
544   0079  90                   MOVX   @R0,A
545   007A  0A                   IN     A,P2
546   007B  53 70                ANL    A,#70H      DSE,DS2E,DS1E
547   007D                       STORE  MODES,0
553   0080  C6 65                JZ     COLDST      WAITING FOR INPUT
554   0082  AD                   MOV    R5,A        TEMP STORAGE OF ENABLES
555   0083  D3 70                XRL    A,#70H      ALL THREE MEAN TESTLOOP
556   0085  C5 93                JZ     TST
557   0087  FD                   MOV    A,R5        GETS ORIG.DATA BACK
558   0088  D3 60                XRL    A,#60H      DSE AND DS2E
559   008A  C6 95                JZ     RA2
560   008C  FD                   MOV    A,R5
561   008D  D3 50                XRL    A,#50H      DSE AND DS1E
562   008F  C6 96                JZ     RA1
563   0091  04 65                JMP    COLDST      IF INVALID INPUTS
564   0093  E4 00     TST        JMP    TESTLP
565   0095  95        RA2        CPL    F0          SETS RANGE2 FLAG
566   0096            RA1        ADC    ECON
576   00A5  03 25                ADD    A,#37
577   00A7  E6 AA                JNC    TMP
578   00A9  27                   CLR    A
```

```
579   00AA  03 06    TMP    ADD    A,#219      RESTORE A TO 219 MAX
580   00AC  A0              MOV    @R0,A       STORE THE NEW ECON
581   00AD  74 8C           CALL   SORT        SQUARE ROOT ROUTINE ON ECON
582   00AF                  STORE  RTECON,0    RA HAS RTECON
```

```
589                    **********************

590                    *

591                    *    NOW COMPUTE IMAX AND VMAX AND VMAXR2

592                    *

593                    **********************

595   00B2  AE              MOV      R6,A

596   00B3  BC 08           MOV      R4,#0D8H

597   00B5  74 50           CALL     MUL

598   00B7  2A              XCH      A,R2

599   00B8                  ROUND    R2

604   00BC                  STORE    IMAX,0     MAX ALLOWABLE CURRENT

610                    *NOTE THAT VMAXR1 AND IMAX ARE REALLY THE SAME

611                    *

612                    *ONE AMPERE IS 200COUNTS, SO IS ONE HUNDRED VOLTS.

613   00BF  FE              MOV      A,R6

614   00C0  77              RR       A

615   00C1  67              RRC      A

616   00C2  53 3F           ANL      A,#3FH

617   00C4  13 00           ADDC     A,#0      ROUNDUP

618   00C6                  STORE    VMAXR2,0   MAX 1/R**2 VOLTAGE

624   00C9  23 0A           MOV      A,#10

625   00CB                  STORE    AFB,0      5 VOLTS IS AFBSET

631   00CE  B6 08           MOV      R0,#DAC    DAC OUTPUT ADDRESS

632   00D0  90              MOVX     @R0,A      WRITE AFB

633   00D1                  PORT     KEY,0

652   00DA                  PORT     RESET,1    RESETNOT NOT ASSERTED

671   00E3                  WAIT     10H

684   00E9  24 00           JMP      GETPWR

685                    *    NOW DO GETPWR FOR CLOSED LOOP
```

LINE  ADD    B1 B2 B3 B4  SSE4 BIPOLAR CONTROL:22 NOVEMBER 1982:CHECKSUM C70

```
687              *************************************
688              *
689              *      GETPWR ROUTINE
690              *
691              *      MEASURES V,1
692              *      COMPUTES Z
693              *      DETERMINES ZRANGE
694              *
695              *      ZRANGE IS A WORD INTERPRETED
696              *      OVOLT,OCCUR,VMAX,IMAX,ZDL,HIZ,MTDZ,LDZ
697              *
698              *      DETERMINES OUTPUT POWER
699              *
700              *      CALLS THE CONTROL LOOP
701              *
702              *************************************

704                         ORG      IOOH
705  0100 27     GETPWR      CLR      A
706  0101 AE                 MOV      R6,A       R6 IS TEMP ZRANGE REGISTER
707  0102                    ADC      VSEN
717  0111 AA                 MOV      R2,A
718  0112 AD                 MOV      R5,A       TEMP SAVE VSEN
719  0113 F2 20              JB7      VFL        IF V>64 VLITE ON
720  0115                    PORT     VFLAG,0    ELSE,LITE OFF
739  011E 24 29              JMP      CKV
740  0120          VFL       PORT     VFLAG,1
759  0129 FD       CKV       MOV      A,R5       GETS V BACK
760  012A 03 37              ADD      A,#55
761  012C E6 32              JNC      CKVMX      CARRY IF OVOLTS
762  012E 2E                 XCH      A,R6
763  012F 43 80              ORL      A,#80H     OVERVOLTS FLAG
764  0131 2E                 XCH      A,R6
765  0132          CKVMX     RECALL   VMAXR1,0
```

| 771 | 0135 | 37 |  | CPL | A | -VMAXR1 |
| 772 | 0136 | 17 |  | INC | A | 25 COMPLEMENT |
| 773 | 0137 | 6A |  | ADD | A,R2 | VSEN-VMAXR1,CARRY IF POSITIVE |
| 774 | 0138 | E6 3E |  | JNC | GET1 |  |
| 775 | 013A | 2E |  | XCH | A,R6 |  |
| 776 | 013B | 43 20 |  | ORL | A,#20H |  |
| 777 | 013D | 2E |  | XCH | A,R6 |  |
| 778 | 013E |  | GET1 | ADC | ISEN |  |
| 788 | 014D | AC |  | MOV | R4,A |  |
| 789 | 014E | F2 5B |  | JB7 | IFL | TURN ON ILITE IF I>640 |
| 790 | 0150 |  |  | PORT | IFLAG,0 | ELSE OFF |
| 809 | 0159 | 24 64 |  | JMP | CKI |  |
| 810 | 015B |  | IFL | PORT | IFLAG,1 |  |
| 829 | 0164 | FC | CKI | MOV | A,R4 | .GETS I BACK |
| 830 | 0165 | 03 37 |  | ADD | A,#55 |  |
| 831 | 0167 | E6 60 |  | JNC | HOUSE | CARRY IF I>1A |
| 832 | 0169 | 2E |  | XCH | A,R6 |  |
| 833 | 016A | 43 40 |  | ORL | A,#40H | OCCURRENT FLAG |
| 834 | 016C | 2E |  | XCH | A,R6 | SAVE ZRANGE |
| 835 | 016D |  | HOUSE | RECALL | IMAX,0 |  |
| 841 | 0170 | 37 |  | CPL | A | -IMAX |
| 842 | 0171 | 17 |  | INC | A | 25 COMPLEMENT |
| 843 | 0172 | 6C |  | ADD | A,R4 | ISEN-IMAX,CARRY IF POSITIVE |
| 844 | 0173 | E6 79 |  | JNC | HOUSE1 |  |
| 845 | 0175 | 2E |  | XCH | A,R6 |  |
| 846 | 0176 | 43 10 |  | ORL | A,#10H | SET IMAX FLAG |
| 847 | 0178 | 2E |  | XCH | A,R6 |  |

LINE  ADDR  B1 B2 B3 B4   SSE4 BIPOLAR CONTROL:22 NOVEMBER 1982:CHECKSUM C70

| | | | | | | |
|---|---|---|---|---|---|---|
| 848 | 0179 | 27 | HOUSE1 | CLR | A | |
| 849 | 017A | 97 | | CLR | C | HOUSEKEEPING |
| 850 | 017B | BB 06 | | MOV | COUNT,#06 | |
| 851 | 017D | 2A | LOOP1 | XCH | A,R2 | |
| 852 | 017E | 67 | | RRC | A | |
| 853 | 017F | EB 7D | | DJNZ | COUNT,LOOP1 | DIVIDES R2 BY 8 |
| 854 | 0181 | 74 66 | | CALL | DIV | R2:A/R4=V/1/8 |
| 855 | 0183 | | | STORE | Z,0 | |
| 861 | 0186 | E6 BE | | JNC | LOOPZ | CARRY IF Z>800 |
| 862 | 0188 | 2E | | XCH | A,R6 | |
| 863 | 0189 | 43 08 | | ORL | A,#8 | HIGH Z FLAG |
| 864 | 018B | 2E | | XCH | A,R6 | |
| 865 | 018C | 24 A6 | | JMP | ZRSTO | |
| 866 | 018E | 03 0F | LOOPZ | ADD | A,#223 | CARRY IF Z>100 OHMS |
| 867 | 0190 | E6 98 | | JNC | MIDZ | |
| 868 | 0192 | 2E | | XCH | A,R6 | |
| 869 | 0193 | 43 04 | | ORL | A,#04 | |
| 870 | 0195 | 2E | | XCH | A,R6 | |
| 871 | 0196 | 24 A6 | | JMP | ZRSTO | |
| 872 | 0198 | 03 0A | MIDZ | ADD | A,#10 | CARRY IF Z>70 OHMS |
| 873 | 019A | E6 A2 | | JNC | LDZ | |
| 874 | 019C | 2E | | XCH | A,R6 | |
| 875 | 019D | 43 02 | | ORL | A,#2 | |
| 876 | 019F | 2E | | XCH | A,R6 | SETS LDZ FLAG |
| 877 | 01A0 | 24 A6 | | JMP | ZRSTO | |
| 878 | 01A2 | 2E | LDZ | XCH | A,R6 | |
| 879 | 01A3 | 43 01 | | ORL | A,#1 | |
| 880 | 01A5 | 2E | | XCH | A,R6 | |
| 881 | 01A6 | | ZRSTO | STORE | ZRANGE,1,R6 | |
| 887 | 01AA | FD | | MOV | A,R5 | VOLTS FOR POWER COMP |
| 888 | 01AB | 74 50 | | CALL | MUL | R4*A GOES TO R2:A |
| 889 | 01AD | 97 | | CLR | C | |
| 890 | 01AE | F7 | | RLC | A | POWER BIT 7 IN CARRY |
| 891 | 01AF | 2A | | XCH | A,R2 | BITS 16-8 IN A |

```
892   0180  F7              RLC     A           2 TIMES POWER
893   0181                  ROUND   R2
898   0185                  STORE   POWER,0
904   0188  44 00           JMP     CNTLP
```

LINE  ADDR  B1 B2 B3 B4  SSE4 BIPOLAR CONTROL:22 NOVEMBER 1982:CHECKSUM C70

```
                        ****************************
                        *
                        *      CNTLP
                        *
                        *      ONCE WE HAVE THE POWER AND IMPEDANCE INFORMATION
                        *      THIS ROUTINE DECIDES WHAT FEEDBACK MODE TO USE.
                        *
                        *****************************

            ORG      200H
   0200     CNTLP    RECALL   ZRANGE,0    OVOLT,OCCUR,VMAX,IMAX,ZDL,HIZ,
                                                      MIDZ,LJZ
   0203 C6 23         JZ       ERROR       ZRANGE NUMBER SHOULDNT BE ZERO
   0205 F2 1F         JB7      OVOLT       V>100
   0207 D2 21         JB6      OCCUR       I>1
   0209 92 27         JB4      CLOZ        1>IMAX
   020B B2 17         JB5      VMAX
   020D 12 27 LOOP2   JB0      CLOZ
   020F 32 33         JB1      CMIDZ       MID RANGE CALC.
   0211 72 25         JB3      ZOL         Z>800
   0213 B6 3B         JF0      HIZR2       Z>100 AND RANGE 2
   0215 44 25         JMP      ZOL         ELSE,Z>100 AND RANGE1
   0217 B6 1B VMAX    JF0      VMAX2       V>VMAX,AND RANGE2
   0219 44 25         JMP      ZOL         HIZ TREATMENT R1
   021B 52 3B VMAX2   JB2      HIZR2       IF 100<Z<800
   021D 44 0D         JMP      LOOP2       BACK UP TO MAIN LOOP
   021F 84 00 OVOLT   JMP      OVOLT4      OVOLT IS ON PAGE 4
   0221 84 47 OCCUR   JMP      OCCUR4
   0223 A4 00 ERROR   JMP      ERROR5      DITTO OCCUR AND ERROR
   0225 44 58 ZOL     JMP      ZOL2        ZOL2 ON PAGE 2
```

LINE  ADDR  B1 B2 B3 B4  SSE4 BIPOLAR CONTROL:22 NOVEMBER 1982:CHECKSUM C70

```
941                       ***********************************
942                       *
943                       *      CLOZ ROUTINE
944                       *
945                       *      COMPUTES F1.CORECT WHERE
946                       *      F1=1 OR 0 AND CORECT IS LESS THAN 1
947                       *
948                       *      ROUTINE USES THE LAST HALF OF THE
949                       *      HIZ ROUTINE FOR COMMONALITY
950                       *
951                       ***********************************

953   0227  A5      CLOZ   CLR     F1          DOWN FLAG
954   0228                 RECALL  ISEN,1,R4
960   022C  AB            MOV     R3,A        SAVE ISEN
961   022D                 RECALL  IMAX,1,R2
967   0231  44 68          JMP     R2ZOL       REST OF ROUTINE IS COMMON
968                       *                    WITH ZOL ROUTINE
```

```
LINE  ADDR  B1 B2 B3 B4  SSE4 BIPOLAR CONTROL:22 NOVEMBER 1982:CHECKSUM C70


                         ***********************

                         *

                         *      ROUTINE MID Z

                         *      SETS PREQ EQUAL TO ECON

                         *      WHICH IS TRUE,MORE OR LESS

                         *

                         ***********************

                         *


      0233         CMIDZ     RECALL     ECON,0

      0236                   STORE      PREQ,0

      0239  84 88           JMP        POWCOM
```

LINE  ADDR  B1 B2 B3 B4  SSE4 BIPOLAR CONTROL:22 NOVEMBER 1982:CHECKSUM C70

```
993                      *********************
994                      *
995                      *                    HIZ
996                      *
997                      *    THIS ROUTINE COMPUTES POWER PREQ
998                      *    FOR THE HIGH IMPEDANCE REGION AND THE
999                      *    1/R**2 MODE
1000                     *
1001                     **************************

1003  023B       HIZR2      RECALL    Z,1,R4
1009  023F  74 50            CALL      MUL          R2=Z**2
1010  0241  F7               RLC       A
1011  0242  27               CLR       A
1012  0243  7A               ADDC      A,R2         A=Z**2+ROUNDUP
1013  0244  AC               MOV       R4,A
1014  0245                   RECALL    ECON,0
1020  0248  97               CLR       C
1021  0249  BA 00            MOV       R2,#00
1022  024B  BB 04            MOV       COUNT,#04
1023  024D  F7       LOOPS   RLC       A
1024  024E  2A               XCH       A,R2
1025  024F  EB 4D            DJNZ      COUNT,LOOPS
1026  0251  74 66            CALL      DIV
1027  0253                   STORE     PREQ,0
1033  0256  84 88            JMP       POWCOM
```

LINE ADDR B1 B2 B3 B4   SSE4 BIPOLAR CONTROL:22 NOVEMBER 1982:CHECKSUM C70

```
1035                    ****************************

1036                    *

1037                    *        ROUTINE ZDL

1038                    *

1039                    *        AIMS FOR A CONSTANT VOLTAGE

1040                    *        VMAXR1 OR VMAXR2 AS SET BY FO

1041                    *        CORECT IS F1.CORECT WHERE F1 IS 1 OR 0

1042                    *        CORECT IS A VALUE LESS THAN 1

1043                    *

1044                    *        CORECT=VREQ/VOUT

1045                    *

1046                    ****************************


1048  0258  A5    ZOL2     CLR      F1            DOWN FLAG

1049  0259              RECALL   VSEN,1,R4

1055  025D  AB           MOV      R3,A          SAVE VSEN

1056  025E              RECALL   VMAXR2,1,R2   IF RANGE 2

1062  0262  B6 68        JFO      R2ZOL

1063  0264              RECALL   VMAXR1,1,R2   IF 1/R RANGE

1069  0268     R2ZOL     SUBTR    R3            A=VMAX-VSEN,CARRY IF POS

1077  0270  E6 74        JNC      NEWSET        IF NEG,DIVIDE R2/R4

1078  0272  B5           CPL      F1            POSITIVE ANSWER,CORECT>1

1079  0273  2A           XCH      A,R2          VMAX-VSEN IN R2

1080  0274  27    NEWSET   CLR      A             R2:00/R4

1081  0275  74 66        CALL     DIV

1082  0277  E6 7B        JNC      CORGET

1083  0279  23 FF        MOV      A,#OFFH       NEED LOTS VOLTS

1084  027B     CORGET    STORE    CORECT,0

1090  027E  C4 00        JMP      AFBADJ
```

```
LINE  ADDR  B1 B2 B3 B4   SSE4 BIPOLAR CONTROL:22 NOVEMBER 1982:CHECKSUM C70

1092                      *HERE IS THE DATA FILE
1093                      *CONTAINING RTECON BASE
1094                      *FOR ALL #S FROM 0 TO 256 IN
1095                      *INCREMENTS OF 4
1096                                   ORG      300H
1097  0300  00                         DB       00
1098  0301  20 2D 37 40                DB       32,45,55,64
1099  0305  48 4E 55 56                DB       72,78,85,91
1100  0309  60 65 6A 6F                DB       96,101,106,111
1101  030D  73 78 7C 80                DB       115,120,124,128
1102  0311  84 88 8B 8F                DB       132,136,139,143
1103  0315  93 96 99 9D                DB       147,150,153,157
1104  0319  A0 A3 A6 A9                DB       160,163,166,169
1105  031D  AC AF B2 B5                DB       172,175,178,181
1106  0321  B8 BB BD C0                DB       184,187,189,192
1107  0325  C3 C5 C8 CA                DB       195,197,200,202
1108  0329  CD CF D2 D4                DB       205,207,210,212
1109  032D  D7 D9 DB DE                DB       215,217,219,222
1110  0331  E0 E2 E5 E7                DB       224,226,229,231
1111  0335  E9 EB ED EF                DB       233,235,237,239
1112  0339  F2 F4 F6 F8                DB       242,244,246,248
1113  033D  FA FC FE FF                DB       250,252,254,255
```

LINE ADDR B1 B2 B3 B4 SSE4 BIPOLAR CONTROL:22 NOVEMBER 1982:CHECKSUM C70

```
                    ************************************

                    *                                  *

                    *    MULTIPLY ROUTINE (8 X 8)      *

                    *                                  *

                    ************************************

                    *                                  *

                    *    IN: A = MULTIPLICAND          *

                    *        R4 = MULTIPLIER           *

                    *                                  *

                    *    OUT: A = LOWER 8 BITS OF PRODUCT  *

                    *         R2 = UPPER 8 BITS OF PRODUCT *

                    *                                  *

                    ************************************


                                ORG        0350H

     0350  BA 00    MUL         MOV        R2,#00

     0352  BB 08                MOV        R3,#8

     0354  12 5E    MPY8LP      JB0        MPY8A

     0356  2A                   XCH        A,R2

     0357  97                   CLR        C

     0358  67                   RRC        A

     0359  2A                   XCH        A,R2

     035A  67                   RRC        A

     035B  EB 54                DJNZ       R3,MPY8LP

     035D  83                   RET

     035E  2A       MPY8A       XCH        A,R2

     035F  6C                   ADD        A,R4

     0360  67                   RRC        A

     0361  2A                   XCH        A,R2

     0362  67                   RRC        A

     0363  EB 54                DJNZ       R3,MPY8LP

     0365  83                   RET
```

LINE  ADDR  B1 B2 B3 B4   SSE4 BIPOLAR CONTROL:22 NOVEMBER 1982:CHECKSUM C70

```
                      *****************************************
                      *                                       *
                      *      DIVIDE ROUTINE (16 X 8 BITS)      *
                      *                                       *
                      *****************************************
                      *                                       *
                      *      IN:  A = (R2:A) / R4             *
                      *                                       *
                      *      OUT: A = QUOTIENT                 *
                      *           R2 = REMAINDER              *
                      *                                       *
                      *****************************************
```

```
      0366  2A        DIV       XCH       A,R2
      0367  8B 08               MOV       R3,#8
      0369  37                  CPL       A
      036A  6C                  ADD       A,R4
      036B  37                  CPL       A
      036C  F6 71               JC        DIVIA
      036E  A7                  CPL       C
      036F  64 8A               JMP       DIVIB
      0371  6C        DIVIA     ADD       A,R4
      0372  97        DIVILP    CLR       C
      0373  2A                  XCH       A,R2
      0374  F7                  RLC       A
      0375  2A                  XCH       A,R2
      0376  F7                  RLC       A
      0377  E6 7E               JNC       DIVIE
      0379  37                  CPL       A
      037A  6C                  ADD       A,R4
      037B  37                  CPL       A
      037C  64 86               JMP       DIVIC
      037E  37        DIVIE     CPL       A
      037F  6C                  ADD       A,R4
```

```
0380  37              CPL     A
0381  E6 86           JNC     DIVIC
0383  6C              ADD     A,R4
0384  64 87           JMP     DIVID
0386  1A      DIVIC   INC     R2
0387  EB 72   DIVID   DJNZ    R3,DIVILP
0389  97              CLR     C
038A  2A      DIVIB   XCH     A,R2
0385  83              RET
```

```
LINE  ADDR  B1 B2 B3 B4   SSE4 BIPOLAR CONTROL:22 NOVEMBER 1982:CHECKSUM C70

1192                        ***********************************
1193                        *
1194                        *       SORT ROUTINE
1195                        *
1196                        *       THIS ROUTINE COMPUTES THE
1197                        *       SQUARE ROOT OF THE VALUE IN THE ACCUMULATOR
1198                        *       BY USING A 6BIT LOOKUP AND LINEAR INTERPOLATION
1199                        *       RETURNS THE VALUE IN THE ACCUMULATOR
1200                        *
1201                        *
1202                        ***********************************


1204  038C  AD       SORT   MOV    R5,A        HOLD X FOR LATER
1205  038D  77              RP     A
1206  038E  77              RP     A
1207  038F  53 3F           ANL    A,#03FH     6BITS OF X
1208  0391  A5              MOV    R6,A
1209  0392  17              INC    A           BASE ADDR +1
1210  0393  E3              MOVP3  A,@A        LOOKUP RTECON BASE+1
1211  0394  2E              XCH    A,R6        R6=BASE RTECON+1
1212  0395  E3              MOVP3  A,@A        RT1FCON
1213  0396  AA              MOV    R2,A        SAVE THE BASE
1214  0397  37              CPL    A           NEGATE BASE
1215  0398  17              INC    A           TWOS COMPLEMENT
1216  0399  6E              ADD    A,R6        BASE+1 MINUS BASE
1217  039A  AC              MOV    R4,A        R4 HAS DIFF
1218  039B  F0              MOV    A,R5        X
1219  039C  53 03           ANL    A,#03H
1220  039E  C5 A5           JZ     LOOPE
1221  03A0  AB              MOV    R3,A
1222  03A1  27              CLR    A
1223  03A2  6C       LOOPF  ADD    A,R4        SUM THE DIFF
1224  03A3  EB A2           DJNZ   R3,LOOPF    1,2,OR 3 TIMES
1225  03A5  77       LOOPE  RR     A
```

```
1226   03A6  67           RRC     A          DIFF/4,CARRY IN CARRY.
1227   03A7  53 3F        ANL     A,#3FH
1228   03A9  7A           ADDC    A,R2       A=RTECON BASE + INTERP.+CARRY
1229   03AA  83           RET
```

EP 0 136 855 B1

LINE  ADDR  B1 B2 B3 B4  SSE4 BIPOLAR CONTROL:22 NOVEMBER 1982:CHECKSUM C70

```
1231                      ********************************
1232                      *
1233                      *      OVOLT ROUTINE
1234                      *
1235                      *      CALLED FROM GETPWR IF V>100 VOLTS
1236                      *      PROCEDURE
1237                      *
1238                      *      SHUTDOWN = 1
1239                      *      WAIT 20 MSEC
1240                      *      AFB=AFB/2
1241                      *      SHUTDOWN = 0
1242                      *      IF VOLD<=VNEW
1243                      *      THEN GOTO ERROR
1244                      *      ELSE
1245                      *      GOTO GETPWR
1246                      *
1247                      ********************************

1249                          ORG     400H
1250 0400         OVOLT4      PORT    RESET,0
1269 0409                     RECALL  AFB,0
1275 040C 97                  CLR     C          CARRY = 0 FOR SHIFT OP.
1276 040D 67                  RRC     A          AFB/2
1277 040E 96 11               JNZ     S+3
1278 0410 17                  INC     A          MAKE SURE AFB>0
1279 0411 B8 0B               MOV     R0,#DAC
1280 0413 90                  MOVX    @R0,A      NEW DAC VOLTAGE
1281 0414                     STORE   AFB,0
1287 0417                     WAIT    20H        SETTLING TIME
1300 041D                     PORT    RESET,1    UNASSERT RESET
1319 0426                     RECALL  VSEN,1,R4
1325 042A                     ADC     VSEN       NEW V IN RA
1335 0439                     SUBTR   R4         A HAS NEWV-OLDV,CARRY IF POS.
1343 0441 E6 45               JNC     S+4
1344 0443 44 23               JMP     ERROR
1345 0445 24 00               JMP     GETPWR     IF OLDV>NEWV, WE ARE OK
```

36

LINE ADDR B1 B2 B3 B4   SSE4 BIPOLAR CONTROL:22 NOVEMBER 1982:CHECKSUM C70

```
1347                    ***********************************
1348                    *
1349                    *       OCCUR ROUTINE
1350                    *
1351                    *       CALLED FROM GETPWR IF 1>1 AMP
1352                    *       PROCEDURE
1353                    *
1354                    *       SHUTDOWN = 1
1355                    *       WAIT 20 MSEC
1356                    *       AFB=AFB/2
1357                    *       SHUTDOWN = 0
1358                    *       IF IOLD<=INEW
1359                    *       THEN GOTO ERROR
1360                    *       ELSE
1361                    *       GOTO GETPWR
1362                    *
1363                    ***********************************
```

```
1365  0447              OCCUR4   PORT    RESET,0
1384  0450  23 0A                MOV     A,#10       SET DACVOLTS TO 5 FOR RESTART
1385  0452  B8 08                MOV     R0,#DAC
1386  0454  90                   MOVX    @R0,4       NEW DAC VOLTAGE
1387  0455                       STORE   AFB,0
1393  045B                       WAIT    60H         SETTLING TIME
1406  045E                       PORT    RESET,1     UNASSERT RESET
1425  0467                       RECALL  ISEN,1,R4
1431  046B                       ADC     ISEN        NEW 1 IN RA
1441  047A                       SUBTR   R4          A HAS NEW I-OLDI,CARRY IF POS.
1449  0482  E6 86                JNC     S+4
1450  0484  44 23                JMP     ERROR
1451  0486  24 00                JMP     GETPWR      IF OLDI>NEWI, WE ARE OK
```

```
LINE  ADDR  B1 B2 B3 B4  SSE4 BIPOLAR CONTROL:22 NOVEMBER 1982:CHECKSUM C70


1453                              LIST    S
1454                    ••••••••••••••••••••••••••••••••••••••••••••
1455                    •
1456                    •     ROUTINE POWCOM
1457                    •
1458                    •     THIS ROUTINE COMPUTES CORECT AS
1459                    •
1460                    •     SORT(PREQ/POWER)-1
1461                    •     IF CORECT>1, F1 IS SET
1462                    •     IF CORECT<1, F1 CLEARED
1463                    •
1464                    •     ROUTINE THEN GOES TO AFBADJ
1465                    •
1466                    •
1467                    ••••••••••••••••••••••••••••••••••••••••••••


1469  048B        POWCOM   RECALL  POWER,1,R4
1475  048C                 RECALL  PREQ,0
1481  048F  B9 04          MOV     COUNT,#4        AF WILL DIVIDE PREQ BY 4
1482  0491  67    LOOPH    RRC     A               BY SHIFTING RIGHT TWICE
1483  0492  2A             XCH     A,R2
1484  0493  EB 91          DJNZ    COUNT,LOOPH
1485  0495  2A             XCH     A,R2
1486  0496  53 C0          ANL     A,#0C0H
1487  0498  2A             XCH     A,R2
1488  0499  53 3F          ANL     A,#3FH          A,R2=00XXXXXX,XX000000
1489  049B  2A             XCH     A,R2            R2,A=00XXXXXX,XX000000
1490  049C  74 66          CALL    DIV
1491  049F  E6 A9          JNC     ROOTER          IF CARRY, NEED LOTS POWER
1492  04A0  A5             CLR     F1
1493  04A1  B5             CPL     F1              F1 SET FOR UP
1494  04A2  23 FF          MOV     A,#0FFH         MAX CORECT
1495  04A4                 STORE   CORECT,0
1501  04A7  C4 00          JMP     AFBADJ
```

```
1502  04A9  74 8C    ROOTER    CALL    SORT      GET PREQ/**POWER**1/2
1503  04AB  97                 CLR     C
1504  04AC  F7                 RLC     A         DOUBLE THE ANSWER
1505  04AD  A5                 CLR     F1        F1=0 FOR CORECT<1
1506  04AE  E6 R1              JNC     CORSET    IF CORECT<=1
1507  04B0  B5                 CPL     F1        IF CORECT>1
1508  04B1        CORSET       STORE   CORECT,0
1514  04B4  04 00              JMP     AFBADJ
```

LINE  ADDR  B1 B2 B3 B4  SSE4 BIPOLAR CONTROL:22 NOVEMBER 1982:CHECKSUM C70

```
1516                      ****************************
1517                      *
1518                      *      ROUTINE AFBADJ
1519                      *
1520                      *      CHECKS THE VALUE OF DWNCNT
1521                      *      IF F1 IS CLEAR (CORRECTION IS DOWN)
1522                      *      INCREMENTS DWNCNT. DWNCNT>4 MEANS ERROR
1523                      *      IF F1 SET, DWNCNT SET TO ZERO.
1524                      *
1525                      *      COMPUTES A NEW AFB BY THE FORMULA
1526                      *
1527                      *      AFB=AFB*(F1)+AFB*CORECT
1528                      *
1529                      *      WHERE CORECT IS LESS THAN ONE
1530                      *      AFB EQUALS ZERO AND AFB<ZERO ARE LOCKED OUT
1531                      *
1532                      *      IF AFB=1 AND F1 IS SET, THE NEW AFB=0.
1533                      ****************************

1535                      ORG    600H
1536  0600        AFBADJ  RECALL  POWER,0     IF POWER LESS THAN 5 WATTS
                                                        (15 COUNTS)
1542  0603 03 F0          ADD     A,#OFOH     WE DONT WANT TO PANIC.
1543  0605 F6 0B          JC      TESTIT      TESTIT CHECKS FOR UP OR DOWN
                                                        CORRECTION
1544  0607 B9 2E          MOV     R1,#DWNCNT  STORAGE ADDRESS FOR CLRCNT
1545  0609 C4 21          JMP     CLRCNT      SETS DWNCNT TO ZERO
1546  060B        TESTIT  RECALL  DWNCNT,0    IS #OF TIMES CORRECT IS <0
1552  060E 76 21          JF1     CLRCNT      IF CORRECTION IS UP
1553  0610 17            INC     A           INCREMENT DWNCNT
1554  0611 A1            MOV     @R1,A       STORE NEW DWNCNT
1555  0612 03 F6          ADD     A,#OF6H     CARRY SET IF DWNCNT>10
1556  0614 F6 1F          JC      ERCALL      IF DWNCNT>10
1557  0616 03 06          ADD     A,#06       CARRY IF DWNCNT>4
```

```
0618  E6 23          JNC     OLDAFB
061A                 RECALL  CORECT,0
061D  F2 23          JB7     OLDAFB          IF CORRECT>0.5, WAIT SOME MORE
061F  A4 00   ERCALL JMP     ERROR5          IF DWNCNT>10 OR CORECT<.5(AND)
                                                 DWNCNT>3

0621  27      CLRCNT CLR     A               DWNCNT SET TO 0 IF CORRECTION UPD
0622  A1             MOV     @R1,A           STORE NEW DWNCNT AND CONTINUE
0623         OLDAFB  PORT    MAXD,0          MAX DRIVE LITE OFF UNLESS
062C                 RECALL  AFB,1,R4
0630  96 34          JNZ     LOOPT
0632  23 01          MOV     A,#1            IF WAS ZERO IS NOW ONE
0634  07     LOOPT   DEC     A               IF AFB=1, A IS NOW 0
0635  96 3E          JNZ     GOON            CONTINUE AS ALWAYS
0637  17             INC     A
0638  17             INC     A               A IS NOW TWO
0639  76 5C          JF1     NEWANS          IF UP FLAG SET.
063B  07             DEC     A               RESTORE AFB TO ONE
063C  C4 5C          JMP     NEWANS
063F         GOON    RECALL  CORECT,0
0641  74 50          CALL    MUL             A:R2=AFB(CORECT)
0643  2A             XCH     A,R2
0644                 ROUND   R2
0648  B5             CPL     F1
0649  76 59          JF1     DOWN
064B  6C             ADD     A,R4            IF F1 WAS SET, ADD AFB TO
                                                 AFB*CORECT

064C  E6 59          JNC     DOWN
064E                 PORT    MAXD,1          MAXIMUM DRIVE LITE
0657  23 FF          MOV     A,#255          IF CARRY,AFB>256 SO SET AFB=255
0659  96 5C   DOWN   JNZ     NEWANS          IF WAS ZERO IS NOW ONE
0658  17             INC     A
```

| LINE | ADD | B1 B2 B3 B4 | SSE4 BIPOLAR CONTROL:22 NOVEMBER 1982:CHECKSUM C70 | | |
|---|---|---|---|---|---|
| 1642 | 065C | | NEWANS | STORE | AFB,0 | |
| 1648 | 065F | B9 08 | | MOV | R1,#DAC | |
| 1649 | 0661 | 91 | | MOVX | @R1,A | |
| 1650 | 0662 | | | WCHDOG | | |
| 1679 | 0672 | | | RECALL | MODES,1,R4 | THIS IS LAST MODE NUMBERS |
| 1685 | 0676 | 0A | | IN | A,P2 | GET NEW KEYING SIGNALS |
| 1686 | 0677 | 53 70 | | ANL | A,#70H | DSE,DS1E,DS2E |
| 1687 | 0679 | 37 | | CPL | A | |
| 1688 | 067A | 6C | | ADD | A,R4 | |
| 1689 | 067B | 37 | | CPL | A | ZERO IF NO CHANGE |
| 1690 | 067C | C6 80 | | JZ | WARMUP | IF NO CHANGE |
| 1691 | 067E | 04 00 | | JMP | INIT | |
| 1692 | 0680 | 24 00 | WARMUP | JMP | GETPWR | AND CONTINUE |

```
1693    ******************************
1694    *
1695    *    KILL ROUTINE
1696    *    SETS KEY OFF
1697    *         SHUTDOWN ON
1698    *         RESET ON
1699    *         AFB TO ZERO
1700    *
1701    *    BRANCH AND HANG
1702    *
1703    ******************************
```

| 1705 | 0682 | | KILL6 | PORT | KEY,1 | |
|---|---|---|---|---|---|
| 1724 | 068B | | | PORT | RESET,0 | |
| 1743 | 0694 | | | WAIT | 5 | |
| 1756 | 069A | | | PORT | RESET,0 | DUMMY INSTR. TO REMOVE SDN INST. |
| 1775 | 06A3 | 27 | | CLR | A | |
| 1776 | 06A4 | B9 08 | | MOV | R1,#DAC | |
| 1777 | 06A6 | | | STORE | AFB,0 | |
| 1783 | 06A9 | 91 | | MOVX | @R1,A | SET DAC VOLTS TO ZERO |
| 1784 | 06AA | | | PORT | ERLITE,1 | TURN ON ERROR LED |
| 1803 | 06B3 | C4 82 | | JMP | KILL6 | BRANCH AND HANG |

LINE ADDR  B1 B2 B3 B4   SSE4 BIPOLAR CONTROL:22 NOVEMBER 1982:CHECKSUM C70

```
1805                    ***********************

1806              *          ERROR ROUTINE

1807              *

1808              *      THIS ROUTINE CHECKS THE CONDITION

1809              *      OF THE DC SWITCHER TO DETERMINE IF

1810              *      THE OUTPUT POWER IS CONTROLLABLE.

1811              *      EXITS TO COLDST IF STILL ON,

1812              *      TO KILL OTHERWISE.

1813              *

1814                    ***************************


1816                       ORG     500H

1817  0500        ERROR5   PORT    KEY,1      KEY OFF

1836  0509                 WCHDOG

1865  0519                 PORT    RESET,1

1884  0522                 PORT    ERLITE,1

1903  052B                 ADC     DCSEN      DCVOLTAGE AT BPLUS

1913  053A  AF             MOV     R6,A       TEMP STORAGE FOR OLD DCSEN

1914  053B  B9 08          MOV     R1,#DAC

1915  053D  27             CLR     A

1916  053E  91             MOVX    @R1,A      AFB EQUALS ZERO

1917  053F  FE      DC50    MOV     A,R6       DCSEN

1918  0540  03 9B          ADD     A,#155

1919  0542  F6 46          JC      DCCHK      CARRY IF VOLTS>50

1920  0544  A4 A1          JMP     COOLDN     IF VOLTS LOW.

1921  0546  BF 0A   DCCHK   MOV     R7,#10     COUNTER RF0.

1922  0548                 ADC     DCSEN

1932  0557  A0             MOV     R5,A       R5 IS NEWVOLTS

1933  0558  37             CPL     A

1934  0559  6E             ADD     A,R6       A=OLD-NEW

1935  055A  F6 7B          JC      RESETCK    CARRY IF OLD>NEW

1936  055C  F0             MOV     A,R5

1937  055D  AE             MOV     R6,A       OLDVOLTS UPDATE

1938  055E                 WAIT    10
```

```
0564                    WCHDOG
0574  EF 46            DJNZ    R7,DCCHK     REPEAT 10 TIMES
0576  A4 0B            JMP     KILL5        IF NO IMPROVEMENT
0578          RESETCK  PORT    RESET,1      RESET OFF
0581                    WAIT    100
0587                    ADC     DCSEN
0596  AD               MOV     R5,A
0597  37               CPL     A
0598  6E               ADD     A,R6         OLD-NEW, CARRY IF POSITIVE
0599  F6 A1            JC      COOLDN       IF NO INCREASE
059B  03 F5            ADD     A,#245       MINUS 10
059D  F6 A1            JC      COOLDN       IF SMALL INCREASE
059F  A4 D8            JMP ·   KILL5        OTHERWISE
05A1  BF 64   COOLDN   MOV     R7,#100      COUNTER REG.
05A3          COOL1    WCHDOG
05B3                    ADC     DCSEN
05C2  03 C8            ADD     A,#200
05C4  E6 00            JNC     COOL2        IF VOLTS.LT.20
05C6                    WAIT    100
05CC  EF A3            DJNZ    R7,COOL1     LOOP 100 TIMES
05CE  A4 D8            JMP     KILL5
05D0          COOL2    PORT    ERLITE,0
05D9  04 65            JMP     COLDST       RESTART IF SWITCHER OK
05DB  04 82   KILL5    JMP     KILL6        PAGING PROBLEMS.
```

```
LINE  ADDR  B1 B2 B3 B4   SSE4 BIPOLAR CONTROL:22 NOVEMBER 1982:CHECKSUM C70


                          ********************************
                          *
                          *       TEST LOOP
                          *
                          *       THIS PROGRAM WAITS 5 SECONDS TO KILL
                          *       THE WATCHDOG TIMER
                          *       THEN READS ONE OF FOUR ADC CHANNELS
                          *       AND DUMPS IT TO THE DAC
                          *       REPEATS FOR EACH CHANNEL FOREVER
                          *
                          ********************************
                                  ORG     0700H
     0700          TESTLP  PORT    KEY,1
     0709                  PORT    RESET,0
     0712                  PORT    SDN,0
     071B  BD 20           MOV     R5,#20H
     071D          LOOP10  WAIT    255
     0723  ED 1D           DJNZ    R5,LOOP10     WAIT 2000H CYCLES
     0725  B8 08           MOV     RD,#08        DAC ADDRESS
     0727  F9      LOOPV   MOV     A,R1          ADC CHANNEL
     0728  17              INC     A
     0729  53 03           ANL     A,#3          CHANNEL * 0 1 2 OR 3
     072B  A9              MOV     R1,A
     072C  BC 00           MOV     R4,#00
     072E  EB 34   ICNT    DJNZ    R3,LOOPU
     0730  1C              INC     R4            HI BYTE OF LOOP COUNTER
     0731  FC              MOV     A,R4
     0732  72 27           JB3     LOOPV         IF COUNTER=8*266
     0734  91      LOOPU   MOVX    @R1,A         ADC CONVERSION
     0735  BD 08           MOV     R5,#8         WAIT FOR EDC TO DROP
     0737  ED 37   LOOPW   DJNZ    R5,LOOPW
     0739  86 3D           JN1     LOOPX         IF EDC DOWN
     073B  E4 39           JMP     S-2
     073D  81      LOOPX   MOVX    A,@R1         GET ADC VALUE
```

```
073E  90          MOVX    @R0,A        DUMP IT TO DAC
073F  0A          IN      A,P2
0740  43 8F       ORL     A,#8FH
0742  37          CPL     A            ZERO IF DSE,DS1E AND DS2E
0743  C6 2E       JZ      ICNT
0745  04 00       JMP     INIT
0747  E4 2E       JMP     ICNT         LOOP THROUGH AGAIN
0749              END
```

ASSEMBLER ERRORS =   0

SSE4 BIPOLAR CONTROL:22 NOVEMBER 1982:CHECKSUM C70

SYMBOL TABLE

| | | | | | | | |
|---|---|---|---|---|---|---|---|
| AFB | 002B | AFBADJ | 0600 | CKI | 0164 | CKV | 0129 |
| CKVMX | 0132 | CLOZ | 0227 | CLRCNT | 0621 | CMIDZ | 0233 |
| CNTLP | 0200 | COLDST | 0065 | COOL1 | 05A3 | COOL2 | 05D0 |
| COOLDN | 05A1 | CORECT | 0029 | CORGET | 027B | CORSET | 0481 |
| DAC | 000B | DC50 | 053F | DCCHK | 0546 | DCSEN | 0023 |
| DIV | 0366 | DIVIA | 0371 | DIVIB | 038A | DIVIC | 0386 |
| DIVID | 0387 | DIVIE | 037E | DIVILP | 0372 | DOG | 0008 |
| DOWN | 0659 | DWNCNT | 002E | ECON | 0020 | ENDINT | 0063 |
| ERCALL | 061F | ERLITE | 0080 | ERROR | 0223 | ERROR5 | 0500 |
| GETI | 013E | GETPWR | 0100 | GOON | 063E | HIZR2 | 023B |
| HOUSE | 016D | HOUSE1 | 0179 | ICNT | 072E | IFL | 015B |
| IFLAG | 0010 | IMAX | 002B | INIT | 0000 | ISEN | 0022 |
| KEY | 0001 | KILL5 | 05DB | KILL6 | 0682 | LOOP1 | 017D |
| LOOP10 | 071D | LOOP2 | 020D | LOOP6 | 0045 | LOOPB | 0061 |
| LOOPE | 03A5 | LOOPF | 03A2 | LOOPH | 0491 | LOOPS | 024D |
| LOOPT | 0634 | LOOPU | 0734 | LOOPV | 0727 | LOOPW | 0737 |
| LOOPX | 073D | LOOPZ | 018E | LDZ | 01A2 | MAXD | 0040 |
| MIDZ | 0198 | MODES | 002F | MPY8A | 035E | MPY8LP | 0354 |
| MUL | 0350 | NEWANS | 065C | NEWSET | 0274 | OCCUR | 0221 |
| OCCUR4 | 0447 | OLDAFB | 0623 | OVOLT | 021F | OVOLT4 | 0400 |
| PIMAGE | 002D | POWCOM | 0488 | POWER | 0026 | PREQ | 0027 |
| RZZOL | 0268 | RA1 | 0096 | RA2 | 0095 | RAMCHK | 0034 |
| RESET | 0002 | RESETC | 0578 | ROM | 005B | ROM1 | 01F4 |
| ROM2 | 02F0 | ROM3 | 03F4 | ROM4 | 04F0 | ROM5 | 05F0 |
| ROM6 | 06F0 | ROM7 | 07F0 | ROMST | 00F0 | ROOTER | 04A9 |
| RTECDN | 002A | SDN | 0004 | SORT | 038C | TESTIT | 060B |
| TESTLP | 0700 | TMP | 00AA | TST | 0093 | VFL | 0120 |
| VFLAG | 0020 | VMAX | 0217 | VMAX2 | 021B | VMAXR1 | 002B |
| VMAXR2 | 002C | VSEN | 0021 | WARMUP | 0680 | Z | 0024 |
| ZOL | 0225 | ZOL2 | 0258 | ZRANGE | 0025 | ZRSTO | 01A6 |

# EP 0 136 855 B1

## Claims

1. An electrosurgical generator comprising:—

a source of electrosurgical RF energy;

means for connecting said source to a patient;

sensor means connected to said RF source for sensing the patient impedance; and

control means connected to said RF source for controlling the output power from said RF source in accordance with said patient impedance, said control means (16) being operable to decrease the output power from said RF soruce (12) with increasing patient impedance, characterized in that, the rate of RF output power decrease is substantially greater than that which would result if the output voltage from the RF source (12) were maintained constant over the range of increasing patient impedance.

2. A generator as in Claim 1 where said control means (16) decreases the output power over the range of increasing patient impedance in accordance with the square of said impedance.

3. A generator as in either one of Claims 1 or 2 wherein said control means (16) includes a microprocessor for adjusting the actual power delivered to said load from said source (12).

4. A generator as in either one of Claims 1 or 2 including means for selecting a nominal power to be delivered to said load where said correction factor also depends on the nominal power to compute the correction factor.

5. A generator as in Claim 4 wherein said nominal power extends from 0 to 70 watts.

6. A generator as in either one of Claims 1 or 2 including a heat sink for said source of electrosurgical energy, the heat sink having a capacity of not more than about four watts.

7. A generator as in either one of Claims 1 or 2 wherein, for all load impedances less than a predetermined value, said correction factor computing means so computes the correction factor that the current through said load remains substantially constant.

8. A generator as in Claim 7 including means for selecting a nominal power to be delivered to said load and where said correction factor equals $I_{MAX}/I_{SEN}$ where $I_{MAX}$ is the current through an impedance of said predetermined value having said nominal power applied thereto and $I_{SEN}$ is the actual current through said load.

9. A generator as in Claim 7 wherein said predetermined value is about 70 ohms.

10. A generator as in either one of Claims 1 or 2 wherein, for all load impedances between predetermined first and second values, said correction factor computing means so computes the correction factor that the power delivered to said load remains substantially constant.

11. A generator as in Claim 10 including means for selecting a nominal power to be delivered to said load and where said correction factor equals $(P_{MAX}/P_{LOAD})$ where $P_{MAX}$ is the nominal power and $P_{LOAD}$ is the actual power delivered to said load.

12. A generator as in Claim 10 wherein said first and second predetermined values are respectively about 70 and 100 ohms.

13. A generator as in either one of Claims 1 or 2 wherein, for all load impedances greater than a predetermined value, said correction factor computing means so computes the correction factor that the voltage across said load remains substantially constant.

14. A generator as in claim 13 including means for selecting a nominal power to be delivered to said load and where said correction factor equals $V_{MAX}/V_{SEN}$ where $V_{MAX}$ is the voltage across an impedance of said predetermined value having said nominal power applied thereto and $V_{SEN}$ is the actual voltage across said load.

15. A generator as in Claim 13 wherein said predetermined value is about 100 ohms.

16. A generator as in either one of Claims 1 or 2 wherein, for all impedances greater than a predetermined value, said correction factor computing means so computes the correction factor that the power delivered to said load is proportional to the inverse of the square of the load impedance.

17. A generator as in Claim 16 including means for selecting a nominal power to be delivered to said load and where said correction factor equals $[(10{,}000 P_{MAX}/Z^2_{LOAD})P_{LOAD}]^{1/2}$ where $P_{MAX}$ is the nominal power, $Z_{LOAD}$ is the load impedance, and $P_{LOAD}$ is the actual power delivered to the load.

18. A generator as in Claim 17 wherein said predetermined value is about 100 ohms.

19. An electrosurgical generator comprising:—

an adjustable source of electrosurgical RF energy;

means for connecting said source to a load including a patient; and

control means for adjusting the power delivered to said load from said source, said control means including means for selecting a plurality of modes of operation of said generator in accordance with said load impedance, said generator being characterised in that said control means (16) includes:—

means of selecting first and second modes of operation of said RF source (12) when said load impedance exceeds a predetermined value;

means for maintaining the voltage across said load substantially constant for all values of said load impedance in excess of said predetermined value in response to said first mode being selected; and

means for reducing the RF output power delivered to said load in accordance with the square of the load impedance for all values of load impedance in excess of said predetermined value in response to said second mode being selected.

48

20. A generator as in Claim 19 wherein said control means (16) includes a microprocessor for adjusting the actual power delivered to said load from said source.

21. A generator as in Claim 19 wherein said means for adjusting the power delivered to said load includes:—

means for computing a correction factor which is a function of the impedance of said load and the selected mode of operation; and

means responsive to the correction factor for adjusting said source of electrosurgical current so that either said first or second mode of operation is implemented depending on the selected mode.

22. A generator as in Claim 20 including means for selecting a nominal power to be delivered to said load and where said correction factor for said first mode of operation equals $V_{MAX}/V_{SEN}$ where $V_{MAX}$ is the voltage across an impedance of said predetermined value having said nominal power applied thereto and $V_{SEN}$ is the actual voltage across said load and where the correction factor for said second mode of operation equals $[(10,000P_{MAX}/Z^2_{LOAD})P_{LOAD}]^{1/2}$ where $P_{MAX}$ is the nominal power, $Z_{LOAD}$ is the load impedance, and $P_{LOAD}$ is the actual power delivered to the load.

23. A generator as in Claim 19 where said predetermined value is about 100 ohms.

## Patentansprüche

1. Elektrochirurgie-Generator mit:
einer Elektrochirurgie-HF-Energiequelle;
Mitteln zum Verbinden dieser Quelle mit einem Patienten;
Sensormitteln, die mit dieser HF-Quelle verbunden sind, zum Erfassen des Patientenwiderstandes; und
Steuermitteln, die mit der HF-Quelle verbunden sind, zum Steuern der Ausgangsleitung der HF-Quelle in Entsprechung zum Patientenwiderstand, wobei die Steuermittel (16) betriebsfähig sind, die Ausgangsleistung der HF-Quelle (12) bei steigendem Patentenwiderstand zu verringern, dadurch gekennzeichnet, daß die Geschwindigkeit der Verringerung der HF-Ausgangsleistung wesentlich größer ist als jene, die sich ergeben würde, wenn die Ausgangsspannung der HF-Quelle (12) über den Bereich des steigenden Patientenwiderstands konstantgehalten würde.

2. Generator nach Anspruch 1, wobei die Steuermittel (16) die Ausgangsleistung über den Bereich des steigenden Patientenwiderstands in Entsprechung zum Quadrat des Widerstands verringern.

3. Generator nach Anspruch 1 oder 2, bei dem die Steuermittel (16) einen Mikroprozessor zum Einstellen der von der Quelle (12) an die Last gelieferten Ist-Leistung enthalten.

4. Generator nach Anspruch 1 oder 2, mit Mitteln zum Auswählen einer an die Last abzugebenden Nennleistung, wobei der Korrekturfaktor auch von der Nennleistung abhängt, um den Korrekturfaktor zu berechnen.

5. Generator nach Anspruch 4, bei dem die Nennleistung von 0 bis 70 Watt reicht.

6. Generator nach Anspruch 1 oder 2, mit einer Wärmesenke für die Elektrochirurgie-Energiequelle, wobei die Wärmesenke eine Kapazität von nicht mehr als ungefähr vier Watt hat.

7. Generator nach Anspruch 1 oder 2, bei dem für alle Lastwiderstände kleiner als ein vorherbestimmter Wert die Korrekturfaktor-Berechnungsmittel den Korrekturfaktor so berechnen, daß der Strom durch die Last im wesentlichen konstant bleibt.

8. Generator nach Anspruch 7, mit Mitteln zum Wählen einer an die Last abzugebenden Nennleistung, und bei dem der Korrekturfaktor gleich $I_{MAX}/I_{SEN}$ ist, wobei $I_{MAX}$ der Strom durch einem Widerstand von vorherbestimmten Wert bei daran angelegter Nennleistung und $I_{SEN}$ der Ist-Strom durch diese Last ist.

9. Generator nach Anspruch 7, bei dem der vorherbestimmte Wert umgefähr 70 Ohm beträgt.

10. Generator nach Anspruch 1 oder 2, bei dem für alle Lastwiderstände zwischen vorherbestimmten ersten und zweiten Werten die Korrekturfaktor-Berechnungsmittel den Korrekturfaktor so berechnen, daß die an die Last abgegebene Leistung im wesentlichen konstant bleibt.

11. Generator nach Anspruch 10, mit Mitteln zum Wählen einer an die Last abzugebenden Nennleistung, und bei dem der Korrekturfaktor gleich $(P_{MAX}/P_{LOAD})^{1/2}$ ist, wobei $P_{MAX}$ die Nennleistung und $P_{LOAD}$ die an die Last abgegebene Ist-Leistung ist.

12. Generator nach Anspruch 10, bei dem der erste bzw. zweite vorherbestimmte Wert ungefähr 70 bzw. 100 Ohm betragen.

13. Generator nach Anspruch 1 oder 2, bei dem für alle Lastwiderstände größer als ein vorherbestimmter Wert die Korrekturfaktor-Berechnungsmittel den Korrekturfaktor so berechnenen, daß die Spannung an der Last im wesentlichen konstant bleibt.

14. Generator nach Anspruch 13, mit Mitteln zum Wählen einer an der Last abzugebenden Nennleistung, und bei dem der Korrekturfaktor gleich $V_{MAX}/V_{SEN}$ ist, wobei $V_{MAX}$ die Spannung am Widerstand mit dem vorherbestimmten Wert, an den die Nennleistung angelegt ist, und $V_{SEN}$ die Ist-Spannung an der Last ist.

15. Generator nach Anspruch 13, bei dem der vorherbestimmte Wert ungefähr 100 Ohm beträgt.

16. Generator nach Anspruch 1 oder 2, bei dem für alle Widerstände größer als ein vorherbestimmter Wert die Korrekturfaktor-Berechnungsmittel der Korrekturfaktor so berechnen, daß die an die Last abgegebene Leistung proportional dem Kehrwert des Quadrates des Lastwiderstands ist.

17. Generator nach Anspruch 16, mit Mitteln zum Wählen einer an die Last abzugebenden Nennleistung, und wobei der Korrekturfaktor gleich $[(10.000 P_{MAX}/Z^2_{LOAD})P_{LOAD}]^{1/2}$ ist, wobei $P_{MAX}$ die Nennleistung ist, $Z_{LOAD}$ der Lastwiderstand ist und $P_{LOAD}$ die an die Last abgegebene Ist-Leistung ist.

18. Generator nach Anspruch 17, bei dem der vorherbestimmte Wert ungefähr 100 Ohm beträgt.

19. Elektrochirurgie-Generator mit:

einer einstellbaren Elektrochirurgie-HF-Energiequelle;

Mitteln zum Verbinden dieser Quelle mit einer einen Patienten einschließenden Last; und

Steuermitteln zum Einstellen der von der Quelle an die Last abgegebenen Leistung, wobei die Steuermittel Mittel zum Wählen einer Mehrzahl von Betriebsarten des Generators in Entsprechung zum Lastwiderstand enthält, welcher Generator dadurch gekennzeichnet, ist, daß die Steuermittel (16) enthalten:

Mittel zum Auswählen einer ersten und einer zweiten Betriebsart der HF-Quelle (12), wenn der Lastwiderstand einen vorherbestimmten Wert übersteigt;

Mittel, um die Spannung an der Last für alle Werte des Lastwiderstands oberhalb des vorherbestimmten Werts in Ansprechen auf die Wahl der ersten Betriebsart im wesentlichen konstant zu halten; und

Mittel zum Verringern der an die Last abgegebenen HF-Ausgangsleistung in Entsprechung zum Quadrat des Lastwiderstands für alle Werte des Lastwiderstands oberhalb des vorherbestimmten Werts in Ansprechen auf die Wahl der zweiten Betriebsart.

20. Generator nach Anspruch 19, bei dem die Steuermittel (16) einen Mikroprozessor zum Einstellen der von der Quelle an die Last abgegebenen Ist-Leistung enthalten.

21. Generator nach Anspruch 19, bei dem die Mittel zum Einstellen der an die Last abgegebenen Leistung enthalten:

Mittel zum Berechnen eines Korrekturfaktors, der eine Funktion des Widerstands der Last und der gewählten Betriebsart ist; und

auf den Korrekturfaktor ansprechende Mittel zum Einstellen der Quelle für Elektrochirurgie-Strom, so daß entweder die erste oder die zweite Betriebsart abhängig von der gewählten Betriebsart implementiert wird.

22. Generator nach Anspruch 20, mit Mitteln zum Wählen einer an die Last abzugebenden Nennleistung, und bei dem der Korrekturfaktor für die erste Betriebsart gleich $V_{MAX}/V_{SEN}$ ist, wobei $V_{MAX}$ die Spannung am Widerstand mit dem vorherbestimmten Wert bei daran angelegter Nennleistung und $V_{SEN}$ die Ist-Spannung an der Last ist, und bei dem der Korrekturfaktor für die zweite Betriebsart gleich $[(10.000 P_{MAX}/Z^2_{LOAD})P_{LOAD}]^{1/2}$ ist, wobei $P_{MAX}$ die Nennleistung ist, $Z_{LOAD}$ der Lastwiderstand ist und $P_{LOAD}$ die an die Last abgegebene Ist-Leistung ist.

23. Generator nach Anspruch 19, bei dem der vorherbestimmte Wert ungefähr 100 Ohm beträgt.

## Revendications

1. Générateur électrochirurgical comprenant:

une source d'énergie radiofréquence électrochirurgicale;

des moyens destinés à connecter ladite source à un patient;

des moyens capteurs connectés à ladite source de radiofréquence pour capter l'impédance du patient; et

des moyens de commande connectés à ladite source de radiofréquence pour commander la puissance de sortie de ladite source de radiofréquence en fonction de ladite impédance du patient, lesdits moyens de commande (16) pouvant être mis en oeuvre de manière à diminuer la puissance de sortie de ladite source (12) de radiofréquence avec un accroissement de l'impédance du patient, caractérisé en ce que le rythme de diminution de la puissance de sortie radiofréquence est sensiblement supérieur à celui qui résulterait si la tension de sortie de la source (12) de radiofréquence était maintenue constant sur l'intervalle d'impédance croissante du patient.

2. Générateur selon la revendication 1, où lesdits moyens (16) de commande diminuent la puissance de sortie sur l'intervalle d'impédance croissante du patient conformément au carré de ladite impédance.

3. Générateur selon l'une des revendications 1 ou 2, dans lequel lesdits moyens de commande (16) comprennent un microprocesseur destiné à régler la puissance réelle fournie à ladite charge par ladite source (12).

4. Générateur selon l'une des revendications 1 ou 2, comprenant des moyens destinés à sélectionner une puissance nominale à fournir à ladite charge, ledit facteur de correction dépendant aussi de la puissance nominale pour calculer le facteur de correction.

5. Générateur selon la revendication 4, dans lequel ladite puissance nominale est comprise entre 0 et 70 watts.

6. Générateur selon l'une des revendications 1 ou 2, comprenant un dissipateur de chaleur pour ladite source d'énergie électrochirurgicale, le dissipateur de chaleur ayant une capacité ne dépassant pas environ 4 watts.

7. Générateur selon l'une des revendications 1 ou 2, dans lequel, pour toutes les impédances de charge inférieures à une valeur prédéterminée, lesdits moyens de calcul du facteur de correction calculent le facteur de correction de manière que le courant traversant ladite charge reste sensiblement constant.

8. Générateur selon la revendication 7, comprenant des moyens destinés à sélectionner une puissance nominale à fournir à ladite charge et dans lequel ledit facteur de correction est égal à $I_{MAX}/I_{SEN}$ où $I_{MAX}$ est le courant tranversant une impédance de ladite valeur prédéterminée à laquelle ladite puissance nominale est appliquée et $I_{SEN}$ est le courant réel traversant ladite charge.

9. Générateur selon la revendication 7, dans lequel ladite valeur prédéterminée est d'environ 70 ohms.

10. Générateur selon l'une des revendications 1 ou 2, dans lequel, pour toutes les impédances de charge comprises entre des première et seconde valeurs prédéterminées, lesdits moyens de calcul du facteur de correction calculent le facteur de correction de façon que la puissance fournie à ladite charge reste sensiblement constante.

11. Générateur selon la revendication 10, comprenant des moyens destinés à sélectionner une puissance nominale à fournir à ladite charge, et dans lequel ledit facteur de correction est égal à ($P_{MAX}/P_{CHARGE}$) où $P_{MAX}$ est la puissance nominale et $P_{CHARGE}$ est la puissance réelle fournie à ladite charge.

12. Générateur selon la revendication 10, dans lequel lesdites première et seconde valeurs prédéterminées sont respectivement d'environ 70 ohms et 100 ohms.

13. Générateur selon l'une des revendications 1 ou 2, dans lequel, pour toutes impédances de charge supérieures à une valeur prédéterminée, lesdits moyens de calcul du facteur de correction calculent le facteur de correction de façon que la tension aux bornes de ladite charge reste sensiblement constante.

14. Générateur selon la revendication 13, comprenant des moyens destinés à sélectionner une puissance nominale à fournir à ladite charge et dans lequel ledit facteur de correction est égal à $V_{MAX}/V_{SEN}$, où $V_{MAX}$ est la tension aux bornes d'une impédance de ladite valeur prédéterminée à laquelle ladite puissance nominale est appliquée et $V_{SEN}$ est la tension réelle aux bornes de ladite charge.

15. Générateur selon la revendication 13, dans lequel ladite valeur prédéterminée est d'environ 100 ohms.

16. Générateur selon l'une des revendications 1 ou 2, dans lequel, pour toutes les impédances supérieures à une valeur prédéterminée, lesdits moyens de calcul du facteur de correction calculent le facteur de correction de manière que la puissance fournie à ladite charge soit proportionnelle à l'inverse du carré de l'impédance de la charge.

17. Générateur selon la revendication 16, comprenant des moyens destinés à sélectionner une puissance nominale à fournir à ladite charge et dans lequel ledit facteur de correction est égal à $[(10\,000 P_{MAX}/Z^2_{CHARGE})P_{CHARGE}]^{1/2}$ où $P_{MAX}$ est la puissance nominale, $Z_{CHARGE}$ est l'impédance de la charge et $P_{CHARGE}$ est la puissance réelle fournie à la charge.

18. Générateur selon la revendication 17, dans lequel ladite valeur prédéterminée est d'environ 100 ohms.

19. Générateur électrochirurgical comprenant:
une source réglable d'énergie radiofréquence électrochirurgicale;
des moyens destinés à connecter ladite source à une charge comprenant un patient; et
des moyens de commande destinés à régler la puissance fournie à ladite charge par ladite source, lesdits moyens de commande comprenant des moyens destinés à sélectionner plusieurs modes de fonctionnement dudit générateur en fonction de ladite impédance de la charge, ledit générateur étant caractérisé en ce que lesdits moyens de commande (16) comprennent:
des moyens destinés à sélectionner des premier et second modes de fonctionnement de ladite source (12) de radiofréquence lorsque ladite impédance de la charge dépasse une valeur prédéterminée;
des moyens destinés à maintenir sensiblement constante la tension aux bornes de ladite charge pour toutes valeurs de ladite impédance de la charge supérieures à ladite valeur prédéterminée en réponse à la sélection dudit premier mode; et
des moyens destinés à réduire la puissance de sortie radiofréquence fournie à ladite charge en fonction du carré de l'impédance de la charge pour toutes valeurs de l'impédance de la charge supérieures à ladite valeur prédéterminée en réponse à la sélection dudit second mode.

20. Générateur selon la revendication 19, dans lequel lesdits moyens (16) de commande comprennent un microprocesseur destiné à régler la puissance réelle fournie à ladite charge par ladite source.

21. Générateur selon la revendication 19, dans lequel lesdits moyens de réglage de la puissance fournie à ladite charge comprennent:
des moyens destinés à calculer un facteur de correction qui est une fonction de l'impédance de ladite charge et du mode de fonctionnement sélectionné; et
des moyens qui, en réponse au facteur de correction, règlent ladite source de courant électrochirurgical afin que ledit premier ou ledit second mode de fonctionnement soit exécuté suivant le mode sélectionné.

22. Générateur selon la revendication 20, comprenant des moyens destinés à sélectionner une puissance nominale à fournir à ladite charge et dans lequel ledit facteur de correction pour ledit premier mode de fonctionnement est égal à $V_{MAX}/V_{SEN}$ où $V_{MAX}$ est la tension aux bornes d'une impédance de ladite

EP 0 136 855 B1

valeur prédéterminée à laquelle ledite puissance nominale est appliquée et $V_{SEN}$ est la tension réelle aux bornes de ladite charge, et dans lequel le facteur de correction pour ledit seconde mode de fonctionnement est égal à $[(10\ 000P_{MAX}/Z^2_{CHARGE})P_{CHARGE}]^{1/2}$ où $P_{MAX}$ est la puissance nominale, $Z_{CHARGE}$ est l'impédance de la charge et $P_{CHARGE}$ est la puissance réelle fournie à la charge.

23. Générateur selon la revendication 19, où ladite valeur prédéterminée est d'environ 100 ohms.

FIG. I

FIG.2

FROM INITIALIZATION

**FIG. 3**

COLDST
$I_{MAX} = (P_{MAX} / 70)^{1/2}$
$V_{MAX} = 10 (P_{MAX})^{1/2}$
26

GETPWR
$P_{LOAD} = V_{SEN} \cdot I_{SEN}$
$Z_{LOAD} = V_{SEN} / I_{SEN}$
SET Z RANGE
28

CNTLP
CHECK FLAGS TO DETERMINE
CONTROL ALGORITHM FOR
LOAD IMPEDANCE AND
SELECTED MODE
30

LOZ
COMPUTE CORRECTION
FOR CONSTANT
CURRENT
CORRECTION =
$I_{MAX} / I_{SEN}$
32

HIZR2
COMPUTE CORRECTION
TO SET POWER =
$P_{MAX} / Z^2$
CORRECTION = $[(10,000\ P_{MAX} / Z^2_{LOAD})\ P_{LOAD}]^{1/2}$
40

MIDZ
COMPUTE CORRECTION
FOR CONSTANT POWER
CORRECTION =
$(P_{MAX} / P_{LOAD})^{1/2}$
36

ZOL
COMPUTE CORRECTION
FOR CONSTANT VOLTAGE
CORRECTION =
$V_{MAX} / V_{SEN}$
38

YES

ENABLES ?
42

NO

AFBADJ
ADJUST CONTROL VOLTAGE
AFB = AFB X
CORRECTION
34

TO INITIALIZATION

3

FIG. 4A

FIG. 4B

EP 0 136 855 B1